# EUROPEAN PATENT APPLICATION

(11) **EP 2 407 173 A1**
(43) Date of publication of application: **18.01.2012**
(21) Application number: 10305777.4
(22) Date of filing: 13.07.2010
(51) Int. Cl.: A61K 38/17, A61K 39/395, C12N 15/11, A61P 35/00

(54) **Method and compositions to induce apoptosis of tumoral cells expressing SHH**

(71) Applicant: Netris Pharma, 69008 Lyon (FR)
(72) Inventor: Mehlen, Patrick, 69740 Genas (FR); Delloye-Bourgeois, Céline, 69008, LYON (FR); Bernet, Agnès, 69740, Genas (FR); Delcros, Jean Guy, 69006, Lyon (FR); Nony, Pascale, 69008, Lyon (FR)
(74) Representative: Colombet, Alain André

(57) **Abstract**

The invention is relative to the use of an effective amount of an agonist of the CDO's apoptotic function for the preparation of a drug to induce cell apoptosis in a patient having tumoral cells bearing CDO and expressing SHH. The agonist may be a CDO fragment, a fusion protein comprising a CDO fragment, an antibody against SHH, or a siRNA which is capable of inhibiting SHH expression. The invention also concerns a pharmaceutical composition containing an agonist of the CDO's apoptotic function or a CDO polypeptide or an antibody specific to CDO, and an agonist of the PTC1's apoptotic function or a PTC1 polypeptide or an antibody specific to PTC1, and a pharmaceutically acceptable excipient or vehicle.

SEQUENCE LISTING

<110>  NETRIS PHARMA

<120>  Method and compositions to induce apoptosis of tumoral cells
       expressing SHH

<130>  BET10L1066

<160>  5

<170>  PatentIn version 3.4

<210>  1
<211>  93
<212>  PRT
<213>  artificial sequence

<220>
<223>  peptide

<400>  1

Arg Phe Ser Ser Arg Pro Ile Thr Gly Pro His Ile Ala Tyr Thr Glu
1               5                   10                  15

Ala Val Ser Asp Thr Gln Ile Met Leu Lys Trp Thr Tyr Ile Pro Ser
            20                  25                  30

Ser Asn Asn Asn Thr Pro Ile Gln Gly Phe Tyr Ile Tyr Tyr Arg Pro
        35                  40                  45

Thr Asp Ser Asp Asn Asp Ser Asp Tyr Lys Arg Asp Val Val Glu Gly
    50                  55                  60

Ser Lys Gln Trp His Met Ile Gly His Leu Gln Pro Glu Thr Ser Tyr
65                  70                  75                  80

Asp Ile Lys Met Gln Cys Phe Asn Glu Gly Gly Glu Ser
                85                  90

<210>  2
<211>  227
<212>  PRT
<213>  artificial sequence

<220>
<223>  peptide

<400>  2

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Glu Glu Met Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  3
<211>  19
<212>  PRT
<213>  artificial sequence

<220>
<223>  peptide

<400>  3

Met Asp Phe Gly Leu Arg Leu Ile Phe Leu Val Leu Val Leu Lys Gly
1               5                   10                  15

Val Leu Cys

<210>  4

<211>  22
<212>  DNA
<213>  artificial sequence

<220>
<223>  primer

<400>  4
gcatctcgtc cttatcaagt gg                                                22


<210>  5
<211>  22
<212>  DNA
<213>  artificial sequence

<220>
<223>  primer

<400>  5
tatggtattc tgctggcgat tc                                                22

## Description

The present invention relates to a method for inducing apoptosis of tumoral cells in a patient having tumoral cells with SHH expression, and to novel apoptosis inducing proteins and pharmaceutical compositions.

During the embryonic development, the morphogen Sonic HedgeHog (SHH) regulates many developmental processes including ventrodorsal patterning of the neural tube, establishment of limb polarity and development of the foregut and axio-cranial skeleton (Jessell, 2000 ; Ingham and McMahon, 2001). In the developing neural tube, SHH is produced by the notochord (NT) and the floor plate (FP), which induces a ventrodorsal gradient of SHH and promotes the differentiation of ventral neurons (Le Douarin and Halpern, 2000). Inhibition of SHH in the neural tube induces a loss of ventral neural tube cell differentiation and massive cell death, supporting its role as a survival factor (Charrier et al., 2001; Thibert et al., 2003). In addition to its expression in FP and NT, SHH is expressed in a precise spatio-temporal pattern in the ectoderm and endoderm of the developing branchial arches. Indeed, during the process of facial skeleton formation, SHH produced by the ventral foregut endoderm constitutes an early signal for jaw development by promoting the survival of facial neural crest cells (NCCs) that colonize the first branchial arc (BA1) and by inducing the patterning of BA1 (Brito et al., 2006). Suppression of SHH ventral foregut endoderm source results in massive apoptosis in the BA1, which is reversed by addition of exogenous recombinant SHH (Brito et al., 2006, 2008).

In adults, SHH signaling is mainly quiescent, being physiologically reactivated only during tissue maintenance and repair. However, SHH signaling appears to be crucial during tumor progression (Berman et al., 2003; Dahmane et al., 1997; Thayer et al., 2003; Watkins et al., 2003). Specifically, abnormal induction of SHH signaling through different means -e.g., downregulation or mutation in SHH receptor or effectors, autocrine or paracrine expression of SHH-, has been associated with many different types of human cancers (Raffel et al., 1997; Taylor et al., 2002; Xie et al., 1998; Yauch et al., 2008). As a consequence, SHH and its downstream signaling currently turn as exciting targets for anti-cancer strategy (Scales and de Sauvage, 2009; Tremblay et al., 2009).

SHH signaling in the target cells has been shown to be mediated mainly via its interaction with the 12-transmembrane receptor Patched 1 (PTC or Ptc1). The binding of SHH to Ptc1 relieves its suppressive effect on Smothened (Smo), an orphan seven-transmembrane receptor that initiates a signaling pathway leading to the activation of the glioma-associated (Gli) family of transcription factors. In absence of SHH, Ptc1 appears to have two effects: first it inhibits Smo activity and second it actively triggers an apoptotic response in specific settings, implicating the cleavage of its intracellular part by caspase. The level of unbound SHH is also tightly dependent on its interaction with several single-transmembrane proteins such as Hedgehog-interacting protein (Hhip1) that was shown to sequester SHH.

SHH has also been described to directly bind several other single-transmembrane proteins such as Cell-adhesion molecule-related/Downregulated by Oncogenes (CDO) and Brother of CDO (BOC), two homologous members of the Neural Cell Adhesion Molecule (N-CAM) family, growth arrest protein (Gas1), and Hedgehog-interacting protein (Hhip1). The latter was shown to tightly regulate the level of unbound SHH as it is known to sequester SHH.

CDO and BOC were described as hypothetical co-receptors of Ptch1 that would positively regulate SHH signaling pathway in cooperation with other SHH-binding proteins such as Gas1 (Martinelli and Fan, 2007; Tenzen et al., 2006; Zhang et al., 2006). CDO is composed of five Ig-like domains and three Fnlll-like domains in the extracellular part followed by a single transmembrane domain and an intracellular domain with no clear homology with other known proteins. CDO was first demonstrated to positively regulate skeletal muscle development, being part of cell surface complexes that implicate other transmembrane receptors and cell-cell adhesion molecules including BOC (Kang et al., 2002; Kang et al., 1998). Surprisingly, mice lacking CDO display microforms of holoprosencephaly (HPE), a developmental defect of the forebrain and midface caused by a failure to delineate the midline, frequently observed in humans and mice presenting disruption of the SHH signaling pathway (Cole and Krauss, 2003; Ming and Muenke, 1998; Wallis and Muenke, 2000). Such phenotypic similarities -even though partial as CDO mutant animals do not fully phenocopy SHH mutant animals- led to the investigation of the possible link between CDO and SHH. CDO third Fnlll-like domain was then shown to directly bind SHH in a calcium-dependent and heparin-independent manner (McLellan et al., 2008). Such an interaction was shown to enhance SHH signaling in specific subregions of SHH expression. The structural domains of CDO necessary for its actions in myogenesis appeared distinct from those implicated in SHH signaling pathway, thus suggesting multiple and independent functions of the receptor (Zhang et al., 2006).

Apart from its role during development, CDO was also described as a potential tumor suppressor whose expression is constitutively downregulated in oncogene-transformed cells. In humans, CDO gene maps to chromosome 11 q23-24, a chromosomic region that frequently displays LOH in multiple cancers such as breast, ovary, colorectal and lung cancer. CDO's potential status of tumor suppressor associated with the survival activity of its ligand SHH is similar to what is observed for several receptors that belong to the so-called dependence receptors family. These receptors share the property of creating cellular state of dependence upon their ligand by inducing apoptosis when unbound by their ligand (Goldschneider and Mehlen, 2010). Such a dependence effect has been hypothesized to be crucial to dictate adequate territories of neural cells migration or localization in the developing nervous system and to eliminate tumor cells that would develop in settings of ligand unavailability (Mehlen and Puisieux, 2006).

These dependence receptors include more than fifteen members that have roles both during development and in tumorigenesis regulation in adulthood. Two of these dependence receptors, DCC and neogenin share structural similarities with CDO (Matsunaga et al., 2004; Mehlen et al., 1998). Together with the survival activity of CDO ligand SHH and CDO's role as a tumor suppressor, we thus investigated whether CDO could act as a dependence receptor.

Tenzen et al. 2006 concern the involvement of CDO and BOC as components and targets of the Hedgehog Signaling Pathway. They show that CDO and BOC bind SHH through a high affinity interaction with a specific fibronectin repeat that is essential for activity. CDO-Fc fusion proteins have been used, comprising the FNIII(3) domain or the entire extracellular domain except for the FNIII(3) domain.

US 7,625,759 concerns a method of using BOC or CDO hedgehog antagonists to inhibit hedgehog signaling, as well as treating and diagnosing disorders relating to hedgehog signaling or over-expression of hedgehog, including cancer, cell proliferative disorders, angiogenesis, neurological disorders, etc. This patent recites "BCO/CDO hedgehog antagonists as being a molecule that antagonizes (e.g. neutralizes or impedes) the native or natural function of a hedgehog polypeptide or hedgehog signaling component, including, for example, (i) by blocking the ability of hedgehog to transduce a signal, such as by blocking a native hedgehog ligand from binding to a receptor, (ii) by blocking a hedgehog receptor (e.g., ptch-1, ptch-2, Smo, etc.) from transmitting to a downstream component in the hedgehog signaling pathway, (iii) by blocking the potentiating or stimulatory activity of a positive regulatory hedgehog signaling component (e.g. CDO), or (iv) by activating or enhancing the repressive activity of a negative regulatory hedgehog signaling component (e.g. BOC). The US patent does mention a "BOC/CDO" hedgehog antagonist which "induces apoptosis" on a glioma tumor cell, without any precision on the mechanism of action that is involved.

The inventors demonstrate herein that CDO displays all the traits to be part of the functional family of dependence receptors. CDO triggers apoptosis in the absence of its ligand SHH, CDO is cleaved at aspartic acid residue(s) by caspase-like protease(s) and CDO triggers apoptosis through a domain exposed after this proteolytic cleavage. These *in vitro* traits have been shown to be common to the vast majority of dependence receptors (Goldschneider and Mehlen, 2010). In addition, the inventors have shown here that these traits confer to the pair SHH/CDO a key regulatory role both during embryonic development - e.g. shown here by the role of SHH in blocking CDO pro-apoptotic activity in the developing neural tube or in the first brachial arc- and cancer progression.

An intriguing consequence of this work is the fact that among the known SHH receptors -i.e., PTC1, HIP, Gas-1, BOC and CDO- at least two of them, PTC1 and CDO, behave as dependence receptors. Thus, SHH mediates cell survival by blocking both CDO and PTC1 dependence receptors pro-apoptotic activity, two receptors that are often expressed in the same cells and have been shown to work together to mediate the classic SHH/Gli signaling (Tenzen et al., 2006; Zhang et al., 2006). Without any intention to be bound by theory, it is deemed that PTC1 and CDO need to be unbound and pro-apoptotic to reach apoptosis. This is also probably what occurs in A549 lung cancer cells which express SHH in an autocrine manner as we have shown here that inactivation of PTC1 or of CDO is sufficient to inhibit the death observed after SHH silencing. In H522 lung cancer cell line, CDO appears to be the only one of the two dependence receptors involved in apoptosis induction as PTC1 silencing has no effect on SHH RNAi-mediated cell death. Moreover the two dependence receptors are not necessarily co-expressed. Indeed, in the developing BA1 model presented here, CDO induces cell death in absence of SHH while PTC1 is barely detected. We show here that CDO expression is decreased in a wide fraction of human cancers and notably in more than 70% of Non-Small Cell Lung Cancer (NSCLC) by a mechanism not yet determined. Moreover we show that CDO expression is a negative constraint for tumor cells, as re-expression of CDO in CDO-low NSCLC cells triggers apoptosis of these cells. Thus, CDO, because of its intrinsic ability to trigger apoptosis in the absence of SHH, appears as a tumor suppressor. It is striking to observe that tumor cells appear to have selected at least two mechanisms to escape dependence on SHH for survival. As described above, one mechanism shown here is the loss of CDO expression. This obviously leads to inactivation of CDO-induced apoptosis no matter SHH is present or not. Here we report that a second mechanism to escape SHH dependence appears to be the autocrine expression of SHH. We show that interference with SHH in SHH-expressing-tumor cells is associated with CDO-dependent apoptosis *in vitro* and tumor growth inhibition *in vivo.* The therapeutic strategy presented here is fundamentally different from the undergoing strategies for SHH-PTC1-Smo-Gli targeted cancer therapy based on inhibitors of either SHH/PTC1 interaction, Smo or Smo-mediated signaling. This strategy is based on the activation of the pro-apoptotic activity of CDO possibly associated with the activation of the pro-apoptotic activity of PTC1. We propose here that a treatment based on inhibition of the interaction between SHH and its dependence receptor CDO could potentially benefit to the large fraction of the patients suffering from lung cancer and possibly other cancers with high SHH levels.

A first object of the invention is thus a method for inducing apoptosis of tumoral cells in a patient having cancer cells bearing CDO receptors and expressing SHH, comprising contacting such cells with an effective amount of an agonist of the CDO's apoptotic function.

A second object of the invention is the use of an effective amount of an agonist of the CDO's apoptotic function for the preparation of a drug to induce tumour cell apoptosis in a patient having cancer cells bearing CDO receptors and expressing SHH.

In an embodiment, the patient has a tumour with autocrine and/or paracrine SHH expression.

In an embodiment, the cancer is a Non-Small Cell Lung Cancer with autocrine and/or paracrine SHH expression.

The term "agonist" is used in the broadest sense, and includes any molecule that partially or fully induces the CDO's apoptotic function or is able to prevent the inhibitory action of SHH on the CDO induced death signaling or apoptosis through binding of SHH to CDO. Preferably this molecule interact with SHH expression or interact with SHH or CDO in a way that impede the binding of SHH to CDO.

The term "with SHH expression" means that the tumour cells express SHH and that this SHH expression is able to ensure the inhibitory action of SHH on the CDO induced death signaling or apoptosis through binding to CDO. SHH may come from autocrine or paracrine secretion in the tumoral cells, which secretion is used by the cells to escape the dependence on SHH for survival.

A third object of the invention is thus a method for inducing apoptosis of tumoral cells in a patient having cancer cells bearing CDO and PTC1 receptors and expressing SHH, comprising contacting such cells with an effective amount of an agonist of the CDO's apoptotic function and an effective amount of an agonist of the PTC1's apoptotic function.

A fourth object of the invention is the use of an effective amount of an agonist of the CDO's apoptotic function and an effective amount of an agonist of the PTC1's apoptotic function for the preparation of a drug to induce tumour cell apoptosis in a patient having cancer cells bearing CDO and PTC1 receptors and expressing SHH.

In an embodiment, the agonist of the CDO's apoptotic function is a CDO polypeptide.

A "CDO polypeptide" includes both native sequence CDO polypeptides, CDO polypeptide variants, and chimeric CDO polypeptides.

"Native sequence CDO polypeptide" comprises a polypeptide having the same amino acid sequence as the corresponding CDO polypeptide found in the human or derived therefrom. The native sequence CDO polypeptide can be natural, i.e. isolated from human, recombinant, i.e. produced by recombinant means, or synthetic, i.e. produced by synthesis.

The native sequence CDO polypeptide encompasses the full-length amino acid sequence of the corresponding CDO polypeptide found in the human or a naturally-occurring truncated or secreted form. It also encompasses a fragment of the full-length amino acid sequence which is capable of binding SHH. It encompasses the extracellular domain of CDO as well as the specific binding domain. In particular, it comprises or is made of the third FnIII-like domain or a fraction of this domain which keep the function of the full-length third Fnlll-like domain.

The "function of the third FnIII-like domain" is the specific binding to SHH.

In an embodiment, the CDO polypeptide comprises or is made of the CDO extracellular domain sequence.

In another embodiment, the CDO polypeptide comprises or is made of the three CDO FnIII (1-3) domain sequence.

In still another embodiment, the CDO polypeptide comprises or is made of the third CDO FnIII (3) domain sequence.

In an embodiment, the CDO polypeptide comprises or is made of the amino acid sequence SEQ ID NO:1.

"CDO polypeptide variants" means a polypeptide which amino acid sequence differs from the corresponding native sequence of CDO polypeptide and which comprises a functional third Fnlll-like domain or a fraction of this domain which keep the function of the full-length third Fnlll-like domain. Such a full-length CDO polypeptide variant or a fragment may have at least about 80, 81, 82, 83, 84, 85, 86, 87, 88, 89, 90, 91, 92, 93, 94, 95, 96, 97, 98 or 99% amino acid sequence identity with the corresponding, either full-length or partial (fragment), native sequence CDO polypeptide, such as the sequence depicted on SEQ ID NO:1.

"Chimeric CDO polypeptides" are CDO polypeptides fused to a heterologous amino acid sequence. The present invention encompasses chimeric CDO polypeptides comprising a fraction of the CDO polypeptide, e.g. the specific binding domain, and supplemental amino acids.

In an embodiment, the chimeric CDO polypeptide is a fusion protein comprising a CDO polypeptide and an immunoglobin domain. "Immunoglobin domain" means a Fc domain, a heavy chain or a light chain.

In a preferred embodiment, the immunoglobin domain is a Fc sequence. It may be in particular a Fc from a human IgG1.

In an embodiment, the IgG Fc fragment comprises or is made of the amino acid sequence SEQ ID NO:2.

In a preferred embodiment, the agonist is made of or comprise a fusion protein comprising SEQ ID NO:1 and SEQ ID NO:2.

Another object of the invention is such a fusion protein containing a CDO fragment which specifically binds to Sonic Hedgehog (SHH), for use as an agonist of the CDO's apoptotic function as a therapeutic agent to induce apoptosis of tumoral cells in a patient.

Another object of the invention is such a fusion protein containing a CDO fragment which specifically binds to Sonic Hedgehog (SHH), for use as an agonist of the CDO's apoptotic function as a therapeutic agent to induce apoptosis of tumoral cells in a patient having a cancer with autocrine or paracrine SHH expression.

Another object of the invention is such a fusion protein containing a CDO fragment which specifically binds to Sonic Hedgehog (SHH), for use as an agonist of the CDO's apoptotic function as a therapeutic agent to induce apoptosis of Non-Small Cell Lung Cancer in a patient.

In an embodiment, the CDO fragment comprises CDO FNIII (3), e.g. as depicted on SEQ ID NO:1.

In another embodiment, the CDO fragment comprises CDO FNIII (1-3).

In still another embodiment, the CDO fragment comprises the CDO extracellular domain.

In an embodiment, the agonist is an antibody which is able to bind to either SHH or CDO in a manner that impedes SHH-CDO binding. The antibody may be specific for the SHH-CDO binding sequence. The antibody may be not specific to this binding sequence but its binding to CDO or SHH impedes the binding of CDO to SHH.

In an embodiment, the agonist is an antibody against SHH specific for the SHH-CDO binding sequence.

In still another embodiment, the agonist is an antibody against CDO specific for the SHH-CDO binding sequence.

"Antibody against SHH specific for the SHH-CDO binding sequence" is used in the broadest sense to designate any antibody that may bind to SHH wherein this binding makes that the binding between SHH and CDO is rendered impossible. In an embodiment, the antibody is specific to the CDO-specific binding amino acid sequence of SHH.

"Antibody" is used in the broadest sense and includes monoclonal antibodies, polyclonal antibodies, single-chain antibodies and antigen binding fragments of these antibodies which exhibit the desired biological activity.

The monoclonal antibodies may be murine, chimeric or humanized.

In still another embodiment, the agonist is a siRNA which is capable of inhibiting SHH expression.

A small interfering RNA or siRNA is a double stranded RNA (dsRNA) that may have from 10 to 50 nucleotides in length and which reduces expression of the target gene. Portions of the first strand are complementary to the target gene, i.e. it has sufficient complementarity to hybridize to the target gene, for example there is at least 80% identity to the target gene or to a portion thereof.

In an embodiment, the agonist of the PTC1's apoptotic function is a PTC1 polypeptide.

A "PTC1 polypeptide" includes both native sequence PTC1 polypeptides, PTC1 polypeptide variants, and chimeric PTC1 polypeptides.

"Native sequence PTC1 polypeptide" comprises a polypeptide having the same amino acid sequence as the corresponding PTC1 polypeptide found in the human or derived therefrom. The native sequence PTC1 polypeptide can be natural, i.e. isolated from human, recombinant, i.e. produced by recombinant means, or synthetic, i.e. produced by synthesis.

The native sequence PTC1 polypeptide encompasses the full-length amino acid sequence of the corresponding PTC1 polypeptide found in the human or a naturally-occurring truncated or secreted form. It also encompasses a fragment of the full-length amino acid sequence which is capable of binding SHH.

"Chimeric PTC1 polypeptides" are PTC1 polypeptides fused to a heterologous amino acid sequence. The present invention encompasses chimeric PTC1 polypeptides comprising a fraction of the PTC1 polypeptide, e.g. the specific binding domain, and supplemental amino acids.

In an embodiment, the chimeric PTC1 polypeptide is a fusion protein comprising a PTC1 polypeptide and an immunoglobin domain. "Immunoglobin domain" means a Fc domain, a heavy chain or a light chain.

In a preferred embodiment, the immunoglobin domain is a Fc sequence. It may be in particular a Fc from a human IgG1.

In an embodiment, the IgG Fc fragment comprises or is made of the amino acid sequence SEQ ID NO:2.

In an embodiment, the agonist is an antibody which is able to bind to either SHH or PTC1 in a manner that impedes SHH-PTC1 binding. The antibody may be specific for the SHH- PTC1 binding sequence. The antibody may be not specific to this binding sequence but its binding to PTC1 or SHH impedes the binding of PTC1 to SHH.

In an embodiment, the agonist is an antibody against SHH specific for the SHH-PTC1 binding sequence.

In still another embodiment, the agonist is an antibody against PTC1 specific for the SHH- PTC1 binding sequence.

"Antibody against SHH specific for the SHH- PTC1 binding sequence" is used in the broadest sense to designate any antibody that may bind to SHH wherein this binding makes that the binding between SHH and PTC1 is rendered impossible. In an embodiment, the antibody is specific to the PTC1-specific binding amino acid sequence of SHH.

"Antibody" is used in the broadest sense and includes monoclonal antibodies, polyclonal antibodies, single-chain antibodies and antigen binding fragments of these antibodies which exhibit the desired biological activity.

The monoclonal antibodies may be murine, chimeric or humanized.

For their use in human treatment, the active principles according to the invention may be included int a pharmaceutical composition that also contains a pharmaceutically acceptable excipient or vehicle. These compositions are thus intended for the uses that have been described above.

The present invention also relates to a pharmaceutical composition or a kit of parts, containing an agonist of the CDO's apoptotic function or a CDO polypeptide or an antibody specific to CDO, and an agonist of the PTC1's apoptotic function or a PTC1 polypeptide or an antibody specific to PTC1, and a pharmaceutically acceptable excipient or vehicle. The composition or kit of part may be for simultaneous, separated or staggered (spaced out over time) administration of the CDO and the PTC1 related active principles.

The present invention also relates to a therapeutic treatment comprising administering a sufficient amount of an agonist according to the invention, or a pharmaceutical composition containing this agonist as an active principle, to a patient in need thereof.

According to one feature, the therapeutic treatment aims at inducing apoptosis through apoptotic function of the CDO, possibly combined to apoptotic function of the PTC1.

By "effective amount" is meant an amount sufficient to achieve a concentration of peptide which is capable of blocking SHH binding to the CDO and/or PTC1 receptor and inducing apoptosis so as to prevent or treat the disease to be treated. Such concentrations can be routinely determined by those of skilled in the art. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, etc. It will also be appreciated by those of stalled in the art that the dosage may be dependent on the stability of the administered peptide.

The treating a cancer is meant a method aiming at curing, improving the condition and/or extending the lifespan of an individual suffering from a cancer.

A "patient in need thereof" is by definition a patient that may benefit from the therapeutic treatment. Typically, the patient in need thereof is a patient having a cancer or tumour, and the tumoral cells express SHH, in particular through autocrine or paracrine secretion. More specifically, the tumoral cells also have the CDO and/or the PTC1 receptor(s).

As the present invention intends to treat those patients having a tumour with CDO and with SHH expression, the invention also relates to a method of treatment with a previous step intended to check whether or not such CDO and/or SHH expression is present in the patient and the treatment with the agonist is done only on patients that respond positive.

Another object of the invention is thus a method of therapeutic treatment comprising at least two phases, wherein the first phase consists in determining whether or not the patient has a tumour with tumoral cells having CDO and/or expressing SHH and the second phase consists in the administration of a sufficient amount of an agonist according to the invention, or a pharmaceutical composition containing this agonist as an active principle, to a patient responding positively to the first phase. In addition to, or in place of CDO detection, PTC1 detection may be done.

According to a feature of the first phase, the SHH expression is autocrine or paracrine.

The present invention has also as an object the use of an agonist according to the invention, or a pharmaceutical composition containing this agonist, as an active principle for treating a patient in need thereof, with such a patient being of the type that has just been defined.

In an embodiment, the method or use provides further for the administration of an agonist of the PTC1 apoptotic function. This administration may be simultaneous or delayed.

The present invention will now be described using example to be taken as non limiting examples.

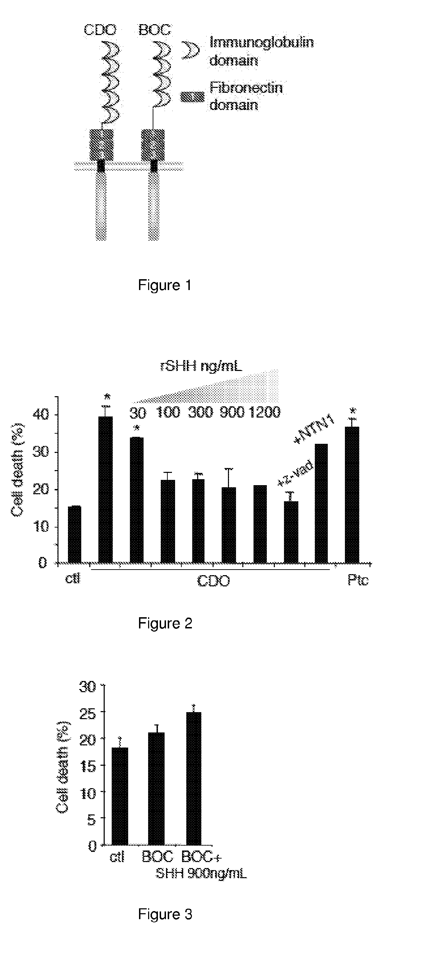

Figure 1: Schematic representation of CDO and BOC protein structural domains.

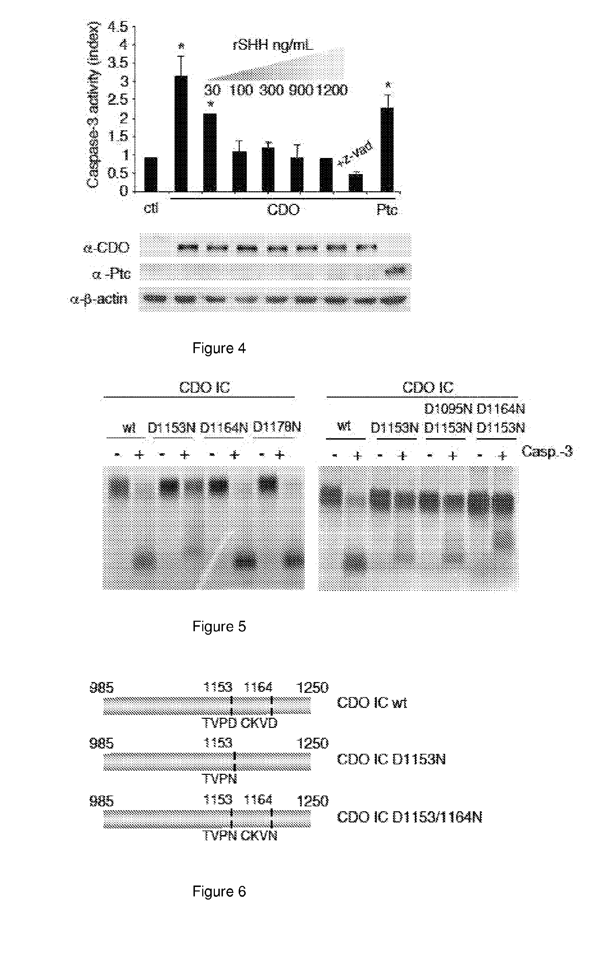

Figure 2-4.: Cell death induction in HEK293T cells was quantified by trypan blue exclusion assay (Fig 2 and 3), caspase-3 activity assay (Fig 4) after transfection with mock, CDO or Ptc1 expressing constructs. Increasing amounts of recombinant SHH added in the cell culture medium are figured above the graphs. Recombinant netrin-1 (NTN1) was added in the culture medium as a negative control. The general caspase inhibitor z-VAD-fmk was used as a control to block apoptotic cell death. In Fig 4, lower panel shows detection of CDO and Ptc1 proteins by Western Blot. Data are means of a minimum of three independent assays. Error bars indicate s.e.m. Statistical treatment of the data was performed using a two-sided Mann-Whitney test compared to mock-transfected condition (* P < 0.05).
Figure 5: Proteolytic cleavage of CDO in its intracellular domain is required for CDO pro-apoptotic activity *in vitro. In vitro*-translated CDO intracellular domains (CDO-IC) wild type (wt) or mutated on one (left panel) or two (right panel) aspartic residues were incubated in the absence or in the presence of recombinant purified active caspase-3. Autoradiographs show a CDO-IC wt cleavage by caspase-3, whereas CDO-IC-D1153N cleavage is strongly decreased and CDO-IC-D1153N-D1164N cleavage is almost completely lost.
Figure 6: Schematic representation of CDO intracellular domain and its different mutant constructs. CDO-IC main (D1153) and secondary (D1164) caspase cleavage sites are presented.

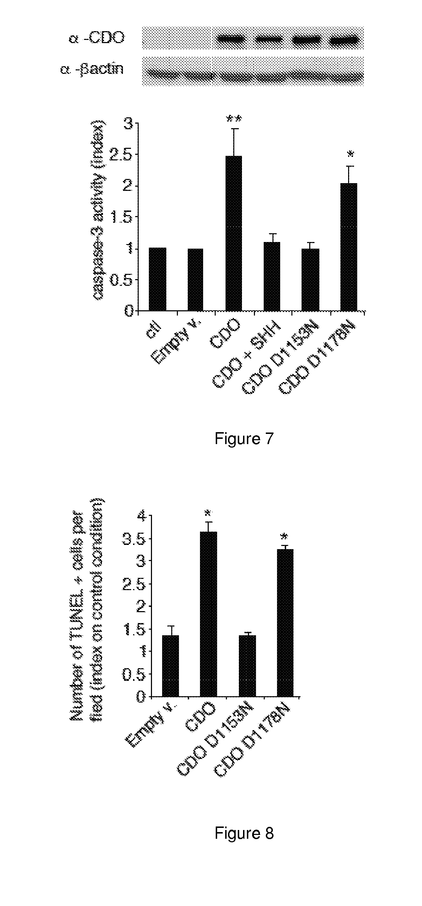

Figures 7-9: Apoptotic cell death induction as measured by caspase-3 activity was quantified in HEK293T cells transfected with wild type (wt) full length or mutated full length CDO constructs. Apoptotic cell death induction as measured by caspase-3 activity was quantified in HEK293T cells transfected with constructs encoding CDO or CDO hypothetical fragments resulting from its cleavage by caspase in D1153 (CDO 1-1153 and CDO 1154-1250). Data are means of a minimum of three independent assays. Error bars indicate s.e.m.. Statistical treatment of the data was performed using a two-sided Mann-Whitney test compared to mock-transfected condition (* P < 0.05; ** P<0.01).

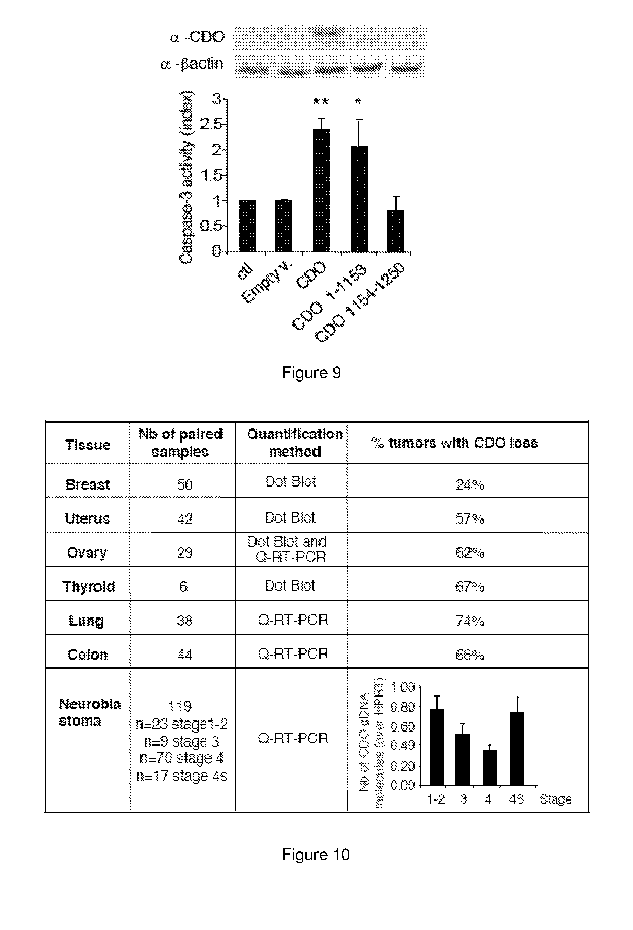

Figure 10: CDO expression is decreased in human cancers. Quantification of CDO expression by Q-RT-PCR and/or by dot blot array in a panel of 328 human matched tumors and paired normal tissues. For each type of tissue, the percent of tumors showing loss of CDO expression as compared to paired normal tissue is indicated. Loss of CDO expression is considered when a more than 2-fold decrease of expression is observed.

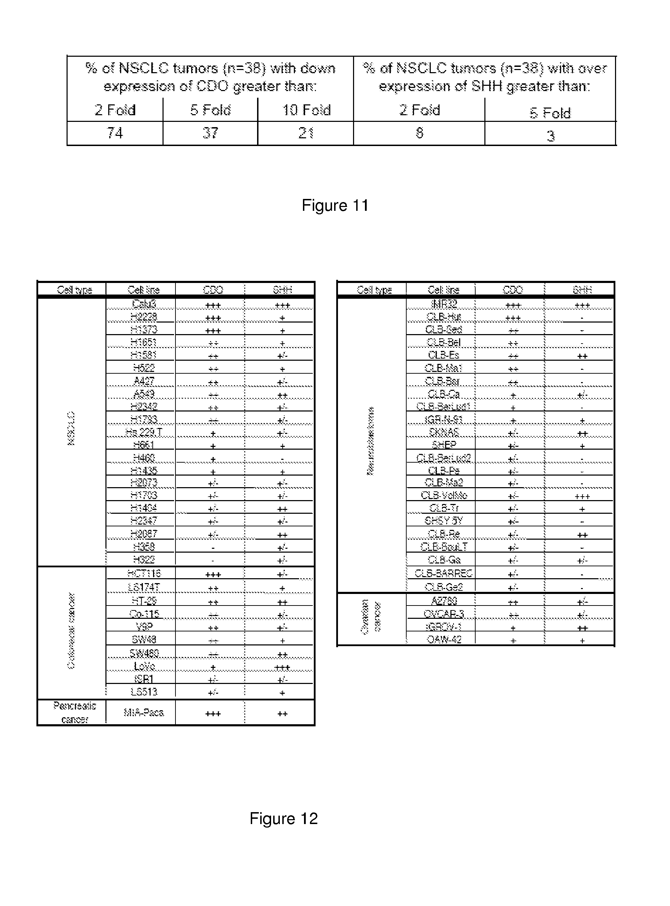

Figure 11: Quantification of CDO expression by Q-RT-PCR in 38 NSCLC and their associated normal tissues. The percentage of patients showing a loss of CDO expression in tumor compared to normal tissue is indicated.
Figure 12: Quantification of CDO and SHH expression by Q-RT-PCR in various cell lines.

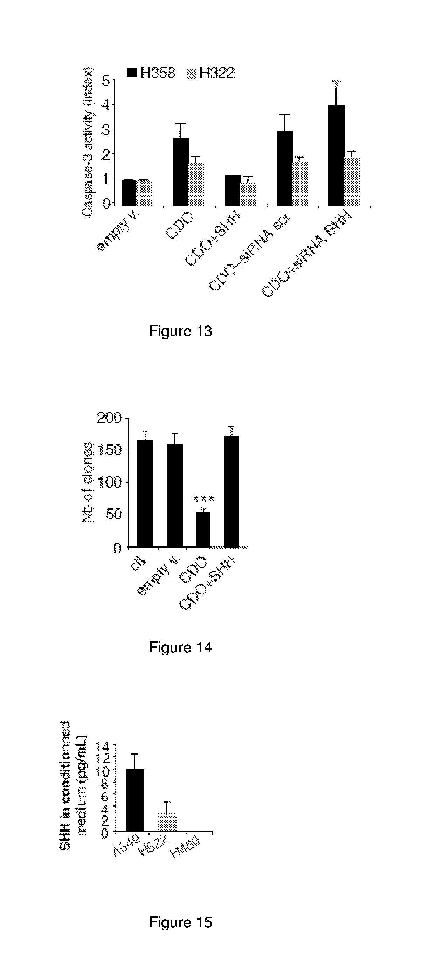

Figure 13: Apoptotic cell death was quantified by caspase-3 assay in H358 and H322 cell lines transfected with CDO encoding construct. SHH availability in the culture medium was modulated either by addition of recombinant SHH (+SHH) in the culture medium or by inhibiting SHH expression via a siRNA approach (+siRNA SHH). Data are means of a minimum of three independent assays. Error bars indicate s.e.m. Statistical treatment of the data was performed using a two-sided Mann-Whitney test compared to mock-transfected condition (* P < 0.05).
Figure 14: Soft agar assay for colony formation was performed on HEK293T cells 14 days after transfection with CDO encoding construct alone or together with siRNA SHH or addition of recombinant SHH. The mean number of clones per dish and per condition is presented. Data are means of a minimum of three independent assays. Error bars indicate s.e.m. Statistical treatment of the data was performed using a two-sided Mann-Whitney test compared to mock-transfected condition (* P < 0.05).
Figure 15: Quantification of endogenous secreted SHH by ELISA assay in A549, H522 and H460 cells culture medium.

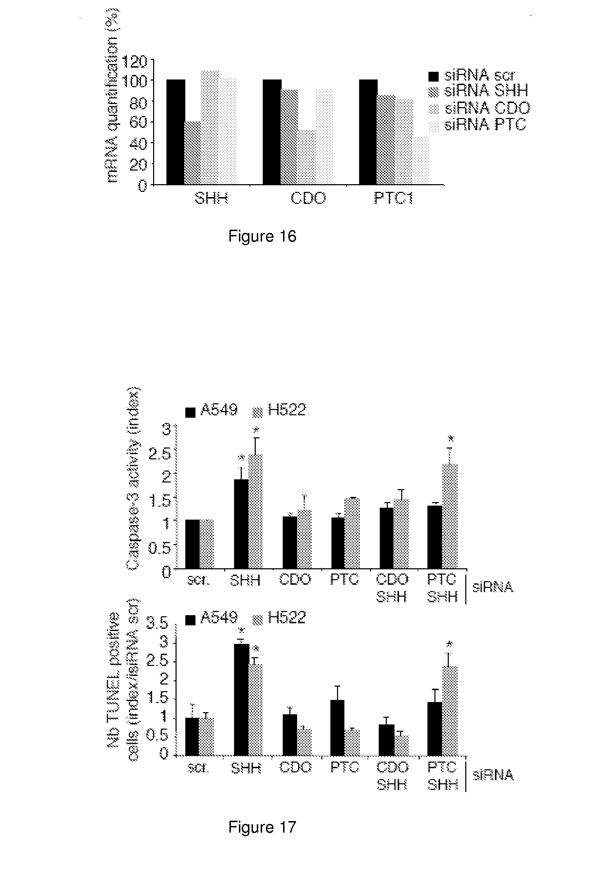

Figure 16: Targeting SHH triggers tumor growth arrest and regression via CDO-induced apoptosis. Quantification of SHH, CDO and PTC1 expression by Q-RT-PCR was performed to check the efficiency and specificity of SHH, CDO and PTC1 siRNAs 24 hours after transfection of A549 cell line.
Figure 17: Apoptotic cell death induction as measured by caspase-3 activity (upper panel) and TUNEL staining (lower panel) was quantified in A549 and H522 cells transfected with SHH siRNA alone or together with CDO siRNA or PTC1 siRNA. Data are means of a minimum of three independent assays. Error bars indicate s.e.m. Statistical treatment of the data was performed using a two-sided Mann-Whitney test compared to scramble siRNA-transfected condition (* P < 0.05).

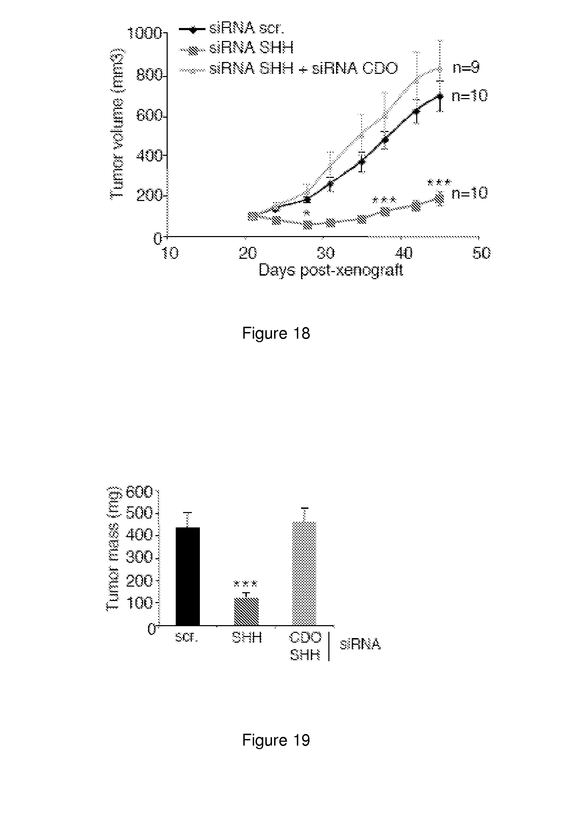

Figures 18-20: Effect of SHH inhibition in NSCLC tumorigenesis. Fig.18: Nude mice were engrafted with A549 cells by subcutaneous injection of 10 millions cells. When the mean tumor volume reached approximately 100 mm³, animals were treated twice a week by intraperitoneal injection of scramble or SHH siRNA alone or in combination with CDO siRNA during 4 weeks. Mean tumor volume and number of animals for each group is indicated.

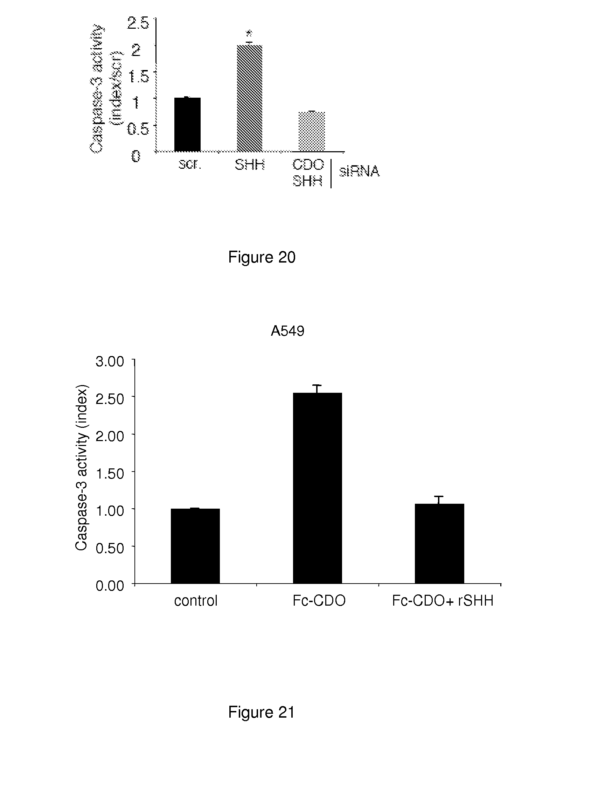

Fig.19: Mean tumor mass of scr siRNA, SHH siRNA or SHH siRNA + CDO siRNAs-treated tumors on day 46, at the end of treatment. Error bars indicate s.e.m. Statistical treatment of the data was performed using a two-sided Mann-Whitney test compared to scramble siRNA-treated condition (* P < 0.05). Fig.20: Apoptosis quantification by caspase-3 activity assay on xenografted tumor lysates analyzed after 1 week of treatment with siRNAs. Error bars indicate s.e.m.
Figure 21: Effect of Fc-CDO on A549 apoptic cell death.

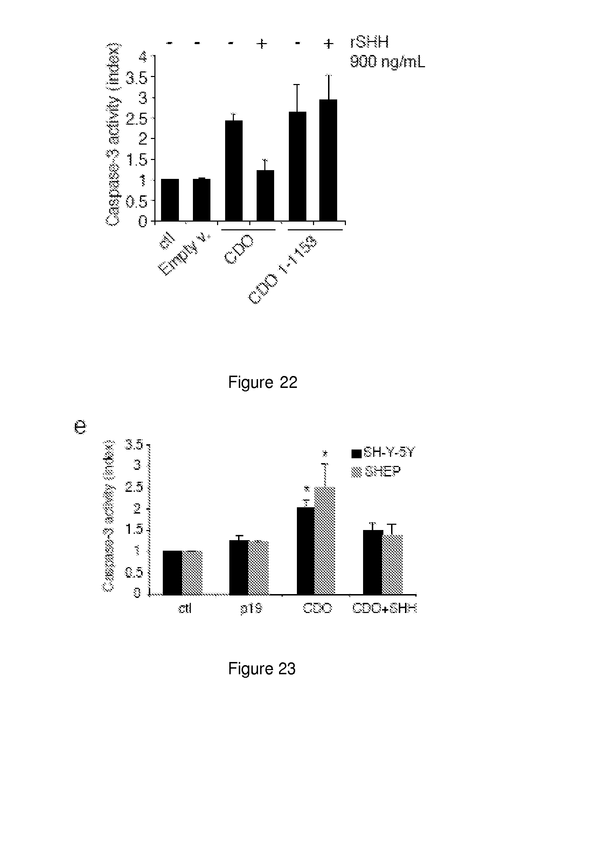

Figure 22. Apoptotic cell death induction as measured by caspase-3 activity was quantified in HEK293T cells transfected with constructs encoding CDO or CDO hypothetical fragment resulting from its cleavage by caspase in D1153 (CDO 1-1153) and treated or not with 900 ng/mL recombinant SHH added in the culture medium. Data are means of a minimum of three independent assays. Error bars indicate s.e.m.
Figure 23. Apoptotic cell death was quantified by caspase-3 assay in SHSY-5Y and SHEP cell lines transfected with CDO encoding construct. SHH availability in the culture medium was modulated by addition of recombinant SHH (+SHH).

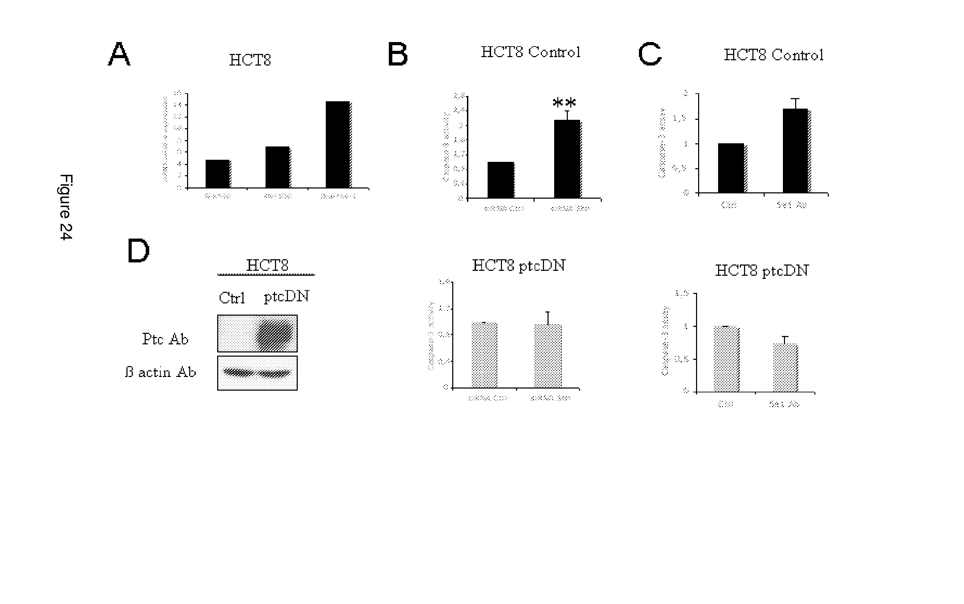

Figure 24 : The interference with SHH triggers Ptc-mediated cell death in HCT8 cells
   (A) Ptc, DRAUFHL2 and SHH mRNA levels in HCT8 cells measured by Q-RT-PCR. (B-C) Cell death was quantified by caspase-3 activity assay in mock transfected control cell and in stably transfected HCT8 with Ptc dominant negative HCT8 cell (HCT8 Ptc DN) 24h after (B) transfection with control siRNA (siRNA Ctrl) or Shh siRNA (siRNA SHH) or (C) SHH-neutralizing 5e1 monoclonal antibody (5e1 Ab). In B-C errors bars indicate SEM. * : p<0.05 ; ** : p<0.01 calculated using two-sided Mann-Whitney test compared with level of control. (D) Analysis of Ptc DN transfected HCT8 cells by Western blot. Ptc dominant negative is expressed (lane ptcDN) in comparison to control cells (lane Ctrl) containing an empty vector.

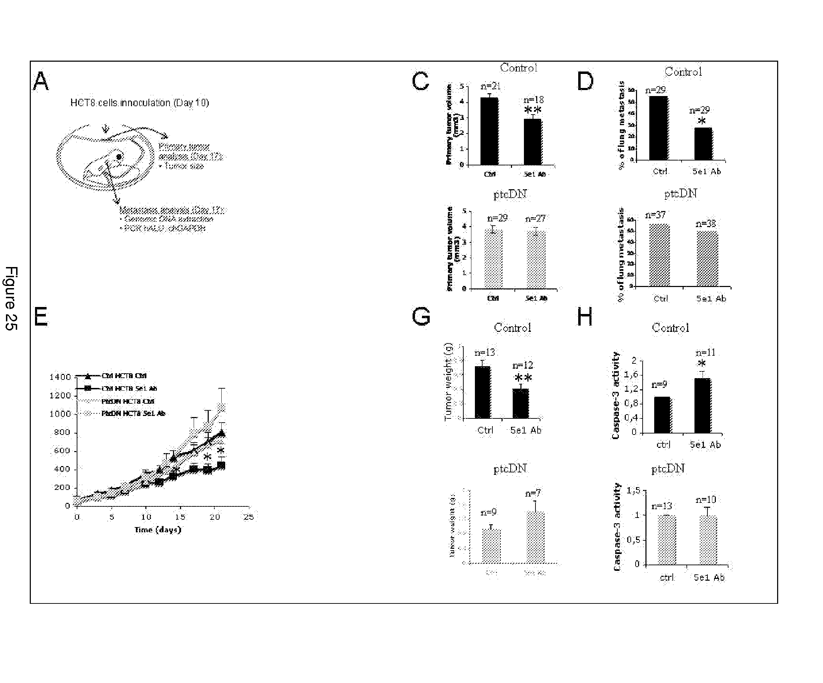

Figure 25: Effect of SHH autocrine loop disruption on HCT8 tumor growth and metastasis in *vivo*
   (A) Schematic representation of the experimental chick model. HCT8 cells were grafted *in* CAM at day 10 and SHH-neutralizing 5e1 monoclonal or IgG control antibodies (10µg/ml) was injected on day 11 and 13. Tumors and lung were harvested on day 17. (C-D) Effect of SHH-neutralizing antibody on primary tumor growth and lung metastasis. (C) Quantitative analysis showing the mean of primary tumor size. (D) Percentage of embryos with lungs invaded by human HCT8 cells. The number of embryos studied in each condition is indicated above the graphs and results are from three independent experiments. In C, errors bars indicate SEM. *: p<0.05; **: p<0.01 calculated using Student t test compared with level of control. In D, *: p<0.05 calculated using a Chi-squared test. (E and G-H) Effect of SHH-neutralizing antibody on HCT8 xenograft tumor growth in mice and on apoptosis. (E) The volume of palpable tumors derived from control or ptcDN HCT8 cells was measured during intratumoral injecion of either 5e1 antibody or control buffer. Mean tumor volume is indicated. (G) The weight of tumors derived from control or ptcDN transfected HCT8 cells was analyzed after 21 days of treatment with 5e1 antibody or control buffer. (H) Quantification of apoptosis by caspase-3 activity assay on xenografts's lysates analyzed after 2 days of intratumoral treatment (after the tumors reached a palpable size). The number of tumor lysates analysed in each condition is indicated above the graphs.. In E, G and H, errors bars indicate SEM. *: p<0.05 ; **: p<0.01 calculated using Student t test compared with level of control.

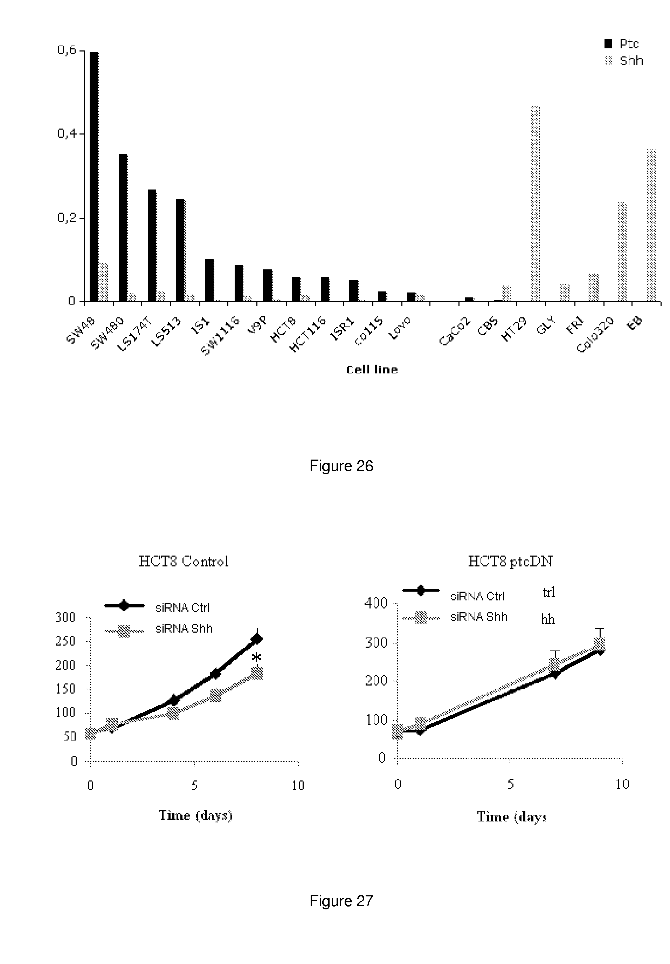

Figure 26: SHH is expressed in a variety of cancer cell lines.
Figure 27:Tumor growth inhibition in SHH siRNA treated HCT8-engrafted mice.
   SHH siRNA versus scramble siRNA effect on HCT8 tumor growth. mock transfected HCT8 or HCT8 Ptc DN cells were subcutaneously engrafted in nude mice. When the engrafted tumors reached 100mm³, mice were three time a week treated intraperitonnaly either with SHH siRNA or with scramble siRNA.

### Example 1: CDO

### Materials and Methods

### Cell line, transfection procedure, reagents:

Human embryonic kidney HEK293T and lung cancer A549 cell lines were cultured in DMEM medium (Gibco®, Invitrogen) containing 10% fetal bovine serum. Human H358, H322 and H522 lung cancer cell lines were cultured in RPMI 1640 Glutamax medium (Gibco®, Invitrogen, Inc, Carlsbad, CA) containing 10% fetal bovine serum. Cell lines were transfected using lipofectamine 2000 reagent (Invitrogen) for small interfering RNA (siRNA) or lipofectamine Plus reagent (Invitrogen) for plasmids. Recombinant human SHH was purchased from R&D system (Minneapolis, MN). 5E1 hybridoma cells producing a Shh-blocking antibody (Developmental Studies Hybridoma Bank) and IgG1 hybridoma cells producing an isotypic unrelated mouse antibody were maintained in Hybri-Care medium (ATCC).

### Human tumors samples and biological annotations:

Following patients' consents, surgical human tumors material was immediately frozen. Human colorectal cancer samples and matched normal tissues (n=44) were provided by the tumor bank at the Hospice Civil de Lyon, fresh tumor tissue being obtained during colorectal surgery prior to any systemic therapy. Human ovary cancer samples and normal tissues (n=15) were obtained from the Biological Resources Center of Centre Léon Bérard, Lyon, France. Human NSCLC samples (selected on the basis of at least 70 % tumor cells in frozen samples) and paired normal tissues were retrieved from the tumor bank of the Pathology Department of the Centre Hospitalier Universitaire, Grenoble, France. Neuroblastoma material (n=119) and annotations were obtained from the Biological Resources Centers of Centre Léon Bérard, Lyon, France and Institut Gustave Roussy, Paris, France. The use of all patient tissue specimens was carried out according to French laws and regulations.

### Plasmid constructs and siRNA:

Mouse CDO fragments were PCR amplified using pBABE-mCDO-Myc (Kang et al., 1998) as a template and cloned in pcDNA3.1 vector using pcDNA™3.1 Directional TOPO® Expression Kit strategy (Invitrogen). A Flag tag was added to each construct. Mouse CDO IC fragments encompass sequences coding for CDO residues Leu⁹⁸⁴ to Thr¹²⁵⁰ (Tenzen et al., 2006 ; see also CDO on Ensembl, under reference ENSMUST 00000119129). Point mutations Asp (GAT or GAC) to Asn (AAT or AAC) were created using the QuickChange site directed mutagenesis strategy (Stratagene) using CDO full length (for cell death assays) or CDO IC (for *in vitro* caspase cleavage assay) constructs as templates. The dominant negative mutant for CDO (CDO-DN) thus corresponds to CDO IC fragment with Asp¹¹⁵⁴ to Asn and Asp¹¹⁶⁴ to Asn point mutations. Dominant negative mutant for PCT1 (pcDNA3.1-PTC1-DN-HA) has been previously described. For *in ovo* chick electroporation, most constructs were based on pMiW vector which was also used as empty vector. Mouse full length CDO was cut from pcDNA3.1-mCDO-Flag and cloned between Hindlll and BgIII sites of pMIW. Mouse CDO fragments cut from pcDNA3.1-CDO-DN-Flag and pcDNA3.1-PTC1-DN-HA were cloned between HindIII and NotI sites of pMIW.

For cell culture use and *in vivo* experiments, human CDO, PCT1 and SHH siRNAs were designed by Santa Cruz (CA) as a pool of 3 to 5 target-specific 20-25nt siRNAs:
- siSHH: sc-29477
- siCDO: sc-60345
- siPTC: sc-36192.

### In vitro translation and caspase-3 cleavage assay:

Plasmids pcDNA3.1-CDO-IC with different point mutations were transcribed using T7 polymerase and then translated using the TNT system (Promega) in the presence of 50µCi [35S]methionine (Perkin Elmer) for 3 h at 30 °C. Translation products were incubated for 2 h in 20mM PIPES pH 7.2, 100mM NaCl, 1% Chaps, 10% sucrose, 10mM dithiothreitol and 0.1mM EDTA, pH 7.2, at 37 °C in the presence of purified active caspase-3. Samples were loaded on a 14% Tris-Glycine acrylamide gel (Invitrogen).

### Cell death assays:

1.8 x 10⁵ cells were grown in serum-poor medium and transfected with plasmids using Lipofectamine Plus Reagent (Invitrogen) or with siRNAs using Lipofectamine 2000 (Invitrogen). Cell death was analysed using trypan blue staining procedures as previously described (Mehlen et al., 1998), 24 hours after transfection. The extent of cell death is presented as the percentage of trypan blue-positive cells in the different cell populations. Apoptosis was monitored by measuring caspase-3 activity as described previously (Mehlen et al., 1998) using Caspase 3/CPP32 Fluorimetric Assay Kit (Gentaur Biovision, Brussel, Belgium) 24 hours after transfection. For detection of DNA fragmentation, treated cells were cytospun 48 hours after transfection and Terminal deoxynucleodityl transferase mediated dUTP-biotin Nick End Labelling (TUNEL) was performed with 300U/mL TUNEL enzyme (300U/mL) and 6 µM biotinylated dUTP (Roche Diagnostics, Meylan, France), as previously described (Ghoumari et al., 2000).

### Soft agar assay for colony formation:

For bottom agar, 2 ml of 1.4% agarose were diluted with 2 ml of 2x DMEM (2x DMEM, 20% foetal bovine serum, 0.2% gentamycin, 500 mg/ml fungizone). A 4-ml volume of bottom agar was plated in a 60-mm tissue culture dish and allowed to harden. Cells were trypsinized and resuspended at 1.10⁶ cells/ml in DMEM medium. The top agar cell suspensions were composed of 100 ml of cell suspension, 1 ml of 2x DMEM, and 1 ml of 0.7% agarose and were overlaid on dishes containing bottom agar. The final plating concentration was 10⁵ cells per dish. 14 days later, clones were fixed with 4% Paraformaldehyde for 5 minutes, stained with 1 ml of 0.005% Crystal Violet for 30 minutes and washed with distilled water.

### Quantitative RT-PCR:

To assay SHH and CDO expression in human tumor and healthy tissues and in human cell lines, total RNA was extracted using the Nucleospin RNAII kit (Macherey-Nagel) and 1 µg was reverse-transcribed using the iScript cDNA Synthesis kit (BioRad). Real-time quantitative RT-PCR was performed on a LightCycler 2.0 apparatus (Roche) using either the Light Cycler FastStart DNA Master SYBERGreen I kit (Roche) for CDO and PTC1 and LightCycler® TaqMan® Master kit (Roche) for SHH. Reaction conditions for all optimal amplifications, as well as primer selection were determined as already described. The ubiquitously expressed human HPRT gene was used as an internal control.

### Dot Blot analysis of CDO expression in human tissues:

CDO gene expression in human tumor and normal paired tissues was monitored by using the Cancer Profiling Array (CLONTECH) following the manufacturer's suggested procedure. CDO probe was prepared by using Amersham Megaprime DNA Labelling System (GE Healthcare) with human full length CDO cDNA as template. The following primers were used: 5'-GCATCTCGTCCTTATCAAGTGG-3' (SEQ ID NO :4) and 5'-TATGGTATTCTGCTGGCGATTC-3' (SEQ ID NO:5). The dot blot was quantified by using the Quantity one 4.6.1 software (Biorad). CDO loss of expression was defined by a fold change (normal versus tumour) of greater than 2.

### Immunohistochemistry and immunoblotting analysis:

For immunohistochemistry on cells, 6.5 10⁴ cells were grown on coverslips. For immunohistochemistry on chick embryos, embryos were embedded in 7.5% gelatine - 0.12M sucrose and 20 µm sections were performed. Slides were fixed in 4% paraformaldehyde and were then incubated at room temperature for two hours with a primary antibody recognizing the human CDO (1:200, R&D systems, Minneapolis, MN), or the FlagM2 tag (1/400, Sigma). After rinsing in Phosphate Buffer Saline, the slides were incubated with an Alexa-488-Donkey or Cy3-Donkey anti-Goat antibody (Molecular Probes), or a Cy5-Donkey anti-Mouse antibody (Jackson ImmunoResearch, Suffolk, UK) (Molecular Probes) respectively. Nuclei were visualized with Hoechst staining. Fluorescence imaging was performed with AxioVision Release 4.6 software.

Immunoblots were performed as already described using anti-CDO (1/2000, R&D Systems), anti-FlagM2 (1/5000, Sigma), anti-HA (1:7500, Sigma) or anti-b-actin (1:1000, Chemicon) primary antibodies.

### SHH ELISA assay:

For SHH determination in cell culture supernatants, white 96-well plate (CORNING) were coated with a monoclonal anti-mouse SHH N-term peptide antibody (MAB4641 - R&D systems), blocked with bovine serum albumin, incubated with the samples, followed by detection of SHH by using a biotinylated anti-mouse Shh antibody (BAM4641 - R&D systems), a streptavidin-peroxidase polymer (S2438 - Sigma) and a chemiluminescent substrate (Pierce ECL Western Blotting Substrate). The luminescence was read on a Tecan Infinite P500 luminometer.

### SHH inhibition in A549-engrafted nude mice:

Seven-week-old (20-22 g body weight) female athymic nu/nu mice were obtained from Charles River animal facility. The mice were housed in sterilized filter-topped cages and maintained in a pathogen-free animal facility. A549 cells were implanted by s.c. injection of 10⁷ cells in 200µL of PBS into the right flank of the mice. When tumors were established (V≈100mm³, approximately 20 days post-injection), mice were treated with scramble siRNA, SHH siRNA and/or CDO siRNA by intra-peritoneal injection of 5 mg total siRNA during 4 weeks, twice a week. Tumor sizes were measured with a caliper. The tumor volume was calculated with the formula v = 0.5 x (length x width²). At the end of the treatment, tumors were harvested, weighted and were embedded in 7.5% gelatine - 0,12M sucrose and sectioned into 20 µm slices. Tumors histology was studied after Hematoxylin-PhloxinB-Safran staining of tumor slides. To measure apoptotic cell death, some tumors were harvested after one week of treatment, immediately frozen and then mechanically lysed in caspase-3 activity lysis buffer. Then, caspase-3 activity was quantified using Caspase 3/CPP32 Fluorimetric Assay Kit (Gentaur Biovision).

### Production of a fusion protein FbnIII-Fc:

Fbnlll-Fc can be expressed efficiently in a variety of host-cells, including Chinese Hamster ovary (CHO) cells, and human embryonic kidney 293 cells. In these systems, the Fc chimera proteins are assembled and secreted into the cell culture medium.

The recombinant expression vector plasmid is transfected into a mammalian host cell line to achieve the expression of the FbnIII-Fc fusion protein. Transfection methods include electroporation, calcium phosphate co-precipitation or transfectant agents such as lipofectamine. Fbnlll-Fc expressing stable clones were selected and identified by checking the expression of the protein by western blot analysis in the medium. For fusion protein batch production, a stable clone is cultured in a serum free culture medium. Fusion protein is purified from the conditioned media on protein-A or protein-G chromatography. Purity is checked by SDS-PAGE analysis. Quantitation of the expressed fusion protein is carried out by anti-human IgG Fc ELISA.

### Exemple of production and purification of a Fc-CDO protein

1. Sequence ID
   a. SEQ ID NO : 1
      CDO Third FbnIII domain
   b. SEQ ID NO : 2
      IgG Fc Fragment
   c. SEQ ID NO : 3
      Peptide Signal : Kappa2 peptide signal
   d. Linker region between CDO Third Fbnlll domain and the IgG Fc Fragment : Gly-Thr.
2. Plasmid construct
   The synthetic gene "kappa2 peptide signal-CDO-Fc" was assembled from synthetic oligonucleotides and cloned into a plasmid allowing protein expression in mammal cells, e.g. the plasmid pFUSE-hIgG1-Fc1 (Invitrogen). After bacteria transformation, its sequence has been verified by sequencing prior to protein production.
3. Transfection/Production method
   FreeStyle HEK 293 cells (Invitrogen) were seeded at 1.10⁶ cells/ml the day of transfection in HEK Freestyle (Invitrogen). Transient transfection was performed using 1.3 µl of 293fectin (Invitrogen) per µg of DNA in Opti-MEM medium. Cell suspension was harvested at day 3 and centrifugated (10 min, 200g, 4°C).
4. Purification method
   Cell supernatant was centrifugated 15 min, 4500g, (4°C) and Tris 500mM pH 8.0 (10xbinding buffer) was added at a final concentration of 10 %. Then it was loaded on Portein G sepharose 4 FF (GE Healthcare) equilibrated with binding buffer (Tris 50mM pH 8.0). Resin was washed with 25 CV (column volume) of binding buffer. Elution was performed with 25 CV of buffer : Glycin 0.1 M pH 2.8. Elution fractions were immediately neutralized by adding 10% of buffer : Tris 1 M pH 9.0.
   Elution fractions were pooled and dialyzed against PBS 1X pH 7.2. Dialyzed pool was concentrated on amicon Da MWCO (Millipore). N-terminal sequencing and Mass spectrometry analysis were performed as quality controls.

### Apoptic cell death using a fusion protein

A549 cells were grown in serum-free medium and treated for 48 hours with 10 µg/mL Fc-CDO with or without 600 ng/mL of recombinant SHH (rSHH). Apoptotic cell death was monitored by measuring caspase-3 activity in each condition.

### Results

### CDO, but not BOC, induces apoptosis in vitro in the absence of SHH:

To determine whether CDO and/or BOC (see graphic representation in Fig.1) are dependence receptors, HEK293T cells were transiently forced to express both receptors. Expression of CDO and BOC could be detected at the plasma membrane (not shown). While, as observed for Ptc, CDO expression was associated with increased cell death as measured by trypan blue exclusion assay (Fig. 2), no significant effect was observed upon,BOC expression (Fig. 3). Different cell lines were assessed to look for an effect of BOC overexpression in cell death but none led to any significant cell death induction, thus disqualifying BOC to be a dependence receptor (not shown).

We thus investigated whether addition of ligand could present CDO-induced cell death. A shown in Figure 2, cell death associated with CDO expression was inhibited in a dose-dependent manner by addition of exogenous recombinant SHH. The protective effect of SHH was specific since the addition of recombinant netrin-1, the ligand of the dependence receptor DCC, had no incidence on CDO associated-cell death. CDO-induced cell death was, at least in part, apoptotic since it was associated with a specific increase in caspase-3 activity (Fig.4) and DNA-fragmentation as measured by TUNEL (Terminal deoxynucleotidyl transferase-mediated deoxyUridine triphosphate Nick End Labeling) staining (not shown). Moreover, such a pro-apoptotic effect was caspase-dependent since the addition of the general caspase inhibitor z-VAD-fmk inhibited CDO-induced cell death (Fig.2-4). CDO behaves as a dependence receptor *in vitro.*

### Proteolytic cleavage, of CDO in its intracellular domain is required for CDO pro-apoptotic activity in vitro:

Dependence receptors share the property of being cleaved in their intracellular part by caspases, a preliminary step required for their pro-apoptotic activity. We thus investigated whether CDO shares this property *in vitro.* As shown in Figures 5 and 6, CDO intracellular domain (CDO-IC, amino acids 985 to 1250) was cleaved *in vitro* when incubated with purified active caspase-3, suggesting the existence of a least one cleavage site in CDO-IC. To determine the putative caspase cleavage site, aspartic acid residues in CDO intracellular domain were successively mutated. While the Asp¹¹⁷⁸ to Asn mutation had no effect on CDO-IC cleavage, mutation of Asp¹¹⁵³ to Asn (CDO-IC D1153N) strongly inhibited cleavage by caspase-3 (Figure 5, right panel). However, this Asp¹¹⁵³ to Asn change revealed the presence of a secondary caspase cleavage site in CDO-IC as incubation of CDO-IC-D1153N with active caspase-3 was associated with the presence of another faint but detectable cleavage fragment. We then mutated different Asp residues in CDO-IC D1153N and assessed cleavage by caspase-3. As shown in Figure 5 (right panel), Asp¹¹⁵³/Asp¹¹⁶⁴ to Asn double mutation almost completely suppressed caspase-3 cleavage indicating that CDO was cleaved *in vitro* by caspase at Asp¹¹⁵³ and Asp¹¹⁶⁴, Asp¹¹⁵³ being the main cleavage site (Fig.6).

To confirm that the CDO cleavage by caspase occurs in cells, GFP-fused CDO mutated or not in D1153N were transfected in HEK293T cells. As shown in Fig.7, CDO cleavage fragment is detected in the cells transfected with wild type CDO but not with CDO D1153N mutant (Fig.7, upper panel). CDO cleavage was strongly inhibited in the presence of the general caspase inhibitor z-VAD-fmk (Fig.7, upper panel). We also looked for cleavage of endogenous CDO *in vivo.* Full length CDO and its putative cleavage fragment were detected in extracts from spinal cords from E12.5 mouse embryos. Treatment of the spinal cords with z-VAD-fmk inhibited the presence of the cleavage fragment, hence confirming CDO cleavage by caspase in vivo (Fig.7, lower panel).

To evaluate the functional relevance of CDO-IC cleavage, the full length CDO D1153N and CDO D1178N (non-caspase cleavage mutation used as control) mutants were transiently expressed in HEK293T cells and cell death was assessed by measuring caspase-3 activity and DNA fragmentation (TUNEL staining). Whereas wild-type CDO and CDO D1178N both triggered apoptotic cell death, CDO D1153N (Fig. 7 and 8) mutant and CDO D1153N/D1164N double mutant (data not shown) failed to induce any increase in caspase-3 activity or DNA fragmentation (Fig.7 and 8). Thus, the cleavage of CDO intracellular domain at Asp¹¹⁵³ is a prerequisite for its pro-apoptotic activity.

In an attempt to identify the region of the CDO intracellular domain implicated in cell death induction -i.e, defined as the Addiction Dependence Domain for dependence receptors-, the pro-apoptotic activity of the CDO fragment potentially released by the caspase cleavage (CDO 1154-1250) or the remaining membrane bound CDO truncated at Asp¹¹⁵³ (CDO 1-1153) was assessed. As shown in Figure 9, expression of CDO 1-1153 fragment induced an increased caspase-3 activation similar to that of full length CDO, whereas the CDO1154-1250 fragment had no effect. Moreover, while SHH addition in the medium suppressed CDO-induced apoptosis, it had no effect on CDO 1-1153 induced apoptosis (Figure 24). Together, these results support the view that CDO addiction dependence domain lies upstream of its caspase cleavage site and that in the absence of SHH, CDO is cleaved by caspase in Asp¹¹⁵³, leading to the exposure of a pro-apoptotic domain located in the N-terminal intracellular region 985-1153.

### CDO acts as a tumor suppressor :

It has been suggested that the pro-apoptotic activity of unbound dependence receptors is a mechanism for eliminating tumor cells that would proliferate in an environment with constant and limited ligand availability or migrate toward tissues devoid of ligand during metastasic dissemination (Mehlen and Guenebeaud, 2009; Mehlen and Puisieux, 2006). Dependence receptors expression is usually decreased or lost in various cancers.(Bernet et al., 2007). It is deemed dependence receptors behave as tumor suppressor. To assess whether CDO expression is affected in human cancers, we first analyzed CDO expression in a panel of 209 human tumors and their corresponding normal tissues either using a dot blot array or Q-RT-PCR. As shown in Fig. 10 CDO expression was decreased in a large fraction of breast, ovary, uterus, thyroid, colon and lung cancers as compared to their normal corresponding tissue.

A marked decrease of CDO expression in epithelial tumor cells as compared to adjacent normal tissues was yet detected by immunohistochemistry in a fraction of human colon adenocarcinoma. We then focused on a pathology in which we could compare within a same tumor type, aggressiveness and prognosis. We thus analyzed by Q-RT-PCR, CDO expression in a panel of 119 neuroblastoma (NB). NB is the most frequent extracranial solid tumor of early childhood and is diagnosed according to 5 stages -i.e., 1,2,3,4 and 4S-. While CDO was detected at a relatively high level in stage 1 or 2 that gather localized NB with low aggressiveness, CDO expression decreased with aggressiveness (Fig. 12). Indeed, stage 4 NB that are highly aggressive and metastatic tumors displayed the lowest level of CDO. Of interest, the stage 4S NB which specifically affect neonates and encompass highly metastatic tumors that yet often spontaneously regress displayed a level of CDO similar to that observed in stage 1-2 (Fig. 12). This did not only occur at the mRNA level but also at the protein level using CDO immunohistochemistry (not shown). This is of particular interest as CDO gene resides at human chromosome 11q24.2, a region very often included in the 11q23-24 deletion detected at high frequency in NB. Moreover, high CDO expression appeared as a good prognostic marker when including all NB tested (overall survival at 10 years of respectively 68% for high CDO tumors and 28% for low CDO tumors, p=0.002) but also more importantly considering metastatic NB only -i.e., stage 4 and stage 4S NB- (overall survival at 10 years of 43% vs 15%, p=0.0077). Thus, high CDO expression in human tumors and more specifically in NB appears as a constraint for tumor progression and aggressiveness.

We then looked for cellular models to study the implication of CDO in tumorigenesis and to do so, we screened a panel of human cancer cell lines for expression of CDO and its ligand SHH (Figure 14). One would expect that, in agreement with the expression of CDO in human tumors, some tumor cells lines have acquired a loss of CDO expression as a survival selective advantage. The neuroblastoma cell lines SHSY-5Y and SHEP, and the lung cancer cell lines H358 and H322 do show weak or no expression of CDO and were thus forced to express CDO. As shown in Figures 23 and 13, CDO expression in these four cell lines was associated with a significant increase in caspase-3 activity, which is reversed by addition of recombinant SHH. This cell death effect observed upon CDO expression was associated with a loss of anchorage-independent growth in soft agar (not shown). Together these data support the view that CDO downregulation observed in a large fraction of cancers allows tumor cell survival in settings of limited SHH availability.

### Targeting SHH inhibits tumor growth via CDO-induced apoptosis:

The therapeutic relevance of CDO as a tumor suppressor via its ability to trigger apoptosis is obviously minimal in the large fraction of cancer with downregulation of CDO. However, it is expected that rather than a loss of CDO expression, some tumors may have selected up-regulation of SHH, which according to the mode of action of dependence receptor, should also be associated with a loss of dependence on SHH availability. Along this line, autocrine or paracrine expression of SHH has been described in different human cancers (Scales and de Sauvage, 2009; Yauch et al., 2008). The general view is that this autocrine or paracrine SHH expression is a selective mechanism which constitutively activates Ptc-Smo signaling (Scales and de Sauvage, 2009; Yauch et al., 2008). We thus investigated whether in the fraction of SHH-expressing tumors, interference with SHH would trigger CDO-mediated tumor cell death. We selected A549 and H522 cell lines which express significant level of CDO mRNA and protein as detected respectively by Q-RT-PCR (Figure 12) and by immunohistochemistry (not shown). These cell lines both expressed a secreted form of SHH that could be quantified by ELISA (respectively, 10.0 ± 2.4 pg and 4.0 ± 1.6 pg per mL of culture medium) thus arguing for an autocrine expression of SHH (Figure 15)..

To investigate the role of this autocrine production of SHH in regulating CDO-induced apoptosis, SHH expression was reduced by a RNA interference-based strategy. Transfection of both A549 and H522 cell lines with SHH, CDO and/or Ptc1 respective siRNAs was associated with a specific and significant decrease of each targeted mRNA (Fig.16). SHH siRNA transfection was associated with an increase in apoptosis monitored by an increase in caspase-3 activity (Fig.17, upper panel) and in the number of TUNEL-labeled cells (Fig. 17, lower panel). Moreover, co-transfection of a CDO siRNA abrogated SHH siRNA induced cell death in both cell lines, thus demonstrating that in SHH/CDO expressing tumor cells, SHH constitutively inhibits CDO-induced apoptosis.

To assess whether this CDO-mediated cell death induction upon SHH interference observed *in vitro* could be translated *in vivo* in a therapeutic perspective, the A549 cell line was engrafted *in nude* mice. Animals with palpable tumors (Tumor volume ≈ 100mm³ Day 21) were treated twice a week by intraperitoneal injection of either scramble siRNA or SHH siRNA alone or in combination with CDO siRNA for 28 days. Tumor volumes were measured over a 46 days period (Fig. 18) and respected tumors were weighted at the end of the treatment (Fig. 19). As shown in Figure 18-19, SHH siRNA was associated with tumor growth inhibition as compared to scramble siRNA-treated mice. Moreover, injection of CDO siRNA blocked the anti-tumor effect observed upon injection of SHH SiRNA, hence demonstrating that in this model the anti-tumor effect associated with SHH inhibition required CDO. To investigate whether the effect on tumor growth could be associated with cell death, we analyzed apoptotic cell death in tumors harvested from engrafted mice treated for one week either with scramble siRNA or with SHH siRNA alone or in combination with CDO siRNA. As shown in Figure 20, caspase-3 activity was significantly increased in tumors from SHH siRNA-treated mice, as compared to tumors from scramble siRNA-treated mice, while such increased apoptosis was not observed in tumors from CDO/SHH siRNAs-treated mice. Moreover, histological analysis of tumors at the end of the treatment revealed a large central area devoid of proliferation tumor cells specifically in SHH siRNA-treated mice but not in SHH/CDO siRNAs-treated animal (not shown). Taken together these data support the view that SHH expression in SHH-high cancer cells is a survival selective advantage to prevent CDO-induced apoptosis.

### Apoptotic cell death using a fusion protein:

Fc-CDO-treated A549 cells show a significant increase in caspase-3 activity which is reversed in presence of recombinant SHH in the medium, which suggests that Fc-CDO-induced cell death depends on SHH binding to its receptor CDO. See Figure 21.

### Example 2: Ptc

The classic morphogen Sonic Hedgehog (SHH), has been shown to play a key role in cancer. Its main receptor Patched (Ptc) has been described as a tumor suppressor and abnormal induction of SHH signaling through different means -e.g., downregulation or mutation in SHH receptor or effectors, autocrine or paracrine expression of SHH-, has been associated with many different types of human cancers. As a consequence, SHH and its downstream signaling currently turn as targets for anti-cancer strategies. These putative therapies are all willing to antagonize the main signaling pathway observed after SHH/Ptc interaction -i.e., Ptc-Smothened (Smo)-Gli-. However, we recently reported that Ptc is not only transducing a signaling through Smo-Gli in the presence of its ligand but is also a dependence receptor. As such, Ptc triggers apoptosis in the absence of SHH, generating a state of dependence on SHH for survival. We show here that the anti-tumor effect observed upon SHH interference is not due to the inhibition of the Ptc-Smo-Gli signaling but rather to the activation of Ptc-induced apoptosis.

SHH signaling in the target cells has been shown to be mediated mainly via its interaction with the twelve-transmembrane receptor Patched 1 (Ptc or PCT1). The binding of SHH to Ptch relieves its suppressive effect on Smoothened (Smo), an orphan seven-transmembrane receptor that initiates a signaling pathway leading to the activation of the glioma-associated (Gli) family of transcription factors.

In adults, SHH signaling is mainly quiescent, being physiologically reactivated only during tissue maintenance and repair. However, SHH signaling appears to be crucial during tumor progression (Dahmane 1997, Watkins 2003, Berman 2003, Thayer 2003). Specifically, abnormal induction of SHH signaling has been associated with many different types of human cancers (Xie 1998, Raffel 1997, Taylor 2002, Yauch 2008). As a consequence, a large spectrum of small molecules or biologics are currently developed to antagonize the SHH-Ptc-Smo-Gli signaling. Two types of therapeutic strategies have been designed: molecules have been engineered to inhibit the intracellular signaling, these compounds includes small molecules, shRNA, monoclonal antibodies mostly targeting Smo or Gli. The second type of approach is related to the fact that SHH is up-regulated in a large fraction of cancer (Yauch 2008) and is based on the interference to SHH production -e.g., oligonucleotides targeting the SHH gene- or to SHH interaction with Ptc -e.g., blocking monoclonal antibody (Scales 2009, Tremblay 2009).

These putative therapeutic strategies are all designed in a "classic" view with Ptc as the trigger of the Smo-Gli signaling upon SHH binding. However, we recently proposed that Ptc is also active in the absence of its ligand and in this setting induces apoptosis. Ptc is thus a dependence receptor (Thibert 2003). Such receptors are two-sided receptors: while they behave as "normal" receptor in the presence of ligand, they induce an active process of apoptotic cell death in the absence of ligand. This dependence on ligand presence is also thought to act as a safeguard mechanism, to prevent tumor cells from developing in settings of ligand unavailability (for reviews Mehlen 2006, Grady 2007). Thus, a tumor cell losing a dependence receptor's pro-apoptotic activity would gain a selective advantage for growth and one of the possible mechanism to achieve this is the up-regulation of the ligand in the tumor environment -i.e. autocrine or paracrine expression- (Fitamant 2008, Delloye-Bourgeois 2009a and b, Bouzas-Rodriguez, Paradisi 2009). Such dependence receptors' ligand up-regulation of ligand is what has been described for SHH in a large fraction of human cancers and our hypothesis is thus that the anti-tumor effect observed upon SHH interference is not due to inactivation of the SHH signaling but to the activation of Ptc pro-apoptotic activity.

To do so, we first investigated whether SHH-expressing tumor cells are surviving because SHH constitutively blocks Ptc-induced apoptosis. We screened a panel of cancer cell lines for expression of SHH, Ptc and the Ptc-mediated death effector DRAL (Mille 2009). Most of the present study will be performed with the colorectal cancer cells HCT8 in which SHH, Ptc and DRAL expression is detected by Q-RT-PCR (Fig.24A). We thus measured HCT8 apoptosis using caspase-3 activity assays, upon silencing of SHH by siRNA or by treatment with the 5e1 antibody. As shown in Fig.24BC, HCT8 cells undergo apoptosis upon SHH interference. Similar results were obtained with other SHH-expressing cells including lung cancer cell A549 or pancreatic carcinoma CAPAN, PANC1 or MiaPaCa2 cells (data not shown). We thus analysed whether this death induction occurs through Ptc pro-apoptotic activity. A mutant of Ptc -i.e., Ptc-7IC-D1392N- (Ptc-DN) was reported to act as a specific dominant negative mutant for Ptc pro-apoptotic activity while this mutant had no effect on the positive signaling Ptc-Smo-Gli ((Thibert 2003, Mille 2009) and Figure 24). We thus generated HCT8 stably expressing Ptc DN (Fig.24D). HCT8 cells expressing Ptc DN now fail to undergo apoptosis, upon silencing of SHH by siRNA or by treatment with the 5E1 antibody, thus supporting the view that HCT8 cells survive in vitro because SHH constitutively inhibits Ptc pro-apoptotic activity.

We thus moved to in vivo tumor models. We first used an avian model that recapitulates tumor progression and dissemination. Grafts of tumor cells in the chorioallantoic membrane (CAM) of 10-day-old chick embryos recapitulates both tumor growth at a primary site - within the CAM- as well as tumor invasion and dissemination at a secondary site - metastasis to the lung ((Stupack 2006) and Fig.25A). HCT8 and HCT8 expressing Ptc DN were then loaded in 10-day-old CAM and embryos were treated on day 11 and day 14 with 5E1 or an unrelated antibody. 17-day-old chicks were then analyzed for primary tumor growth and metastasis to the lung. As shown in Fig.25CD, in HCT8 cells grafted CAM, treatment with the 5E1 antibody significantly reduced primary tumor size and lung metastasis, while an unrelated isotopic antibody had no effect. However when HCT8 Ptc DN cells were grafted instead of mock transfected, 5E1 antibody has no effect on both primary tumor or lung metastasis (Fig.25CD).

The proof-of concept that SHH interference is associated with anti-tumor effect in vivo was provided on classic xenografts of SHH expressing tumor cells in nude mice, We thus subcutaneously engrafted mock transfected HCT8 or HCT8 Ptc DN cells in nude mice. When the engrafted tumors reached 100mm³, mice were three time a week treated intraperitonnaly either with SHH siRNA or with scramble siRNA as successfully performed previously (Delloye-Bourgeois et al. 2009a and 2009b). As shown in Fig.25, the growth of tumors of from mock transfected HCT8 engrafted mice treated with SHH siRNA was significantly reduced compared to the ones engrafted in scramble treated mice. No such effect was observed when Ptc DN expressing HCT8 cells were engrafted (Fig.25). Similar experiment was performed using 5E1 antibody treatment instead of siRNA. As shown in Fig.25E, 5E1 antibody treatment significantly reduces tumor growth on mock transfected HCT8. This tumor growth inhibition is associated with a significant reduction of the net tumor weight at the end of treatment protocol (Fig.25G). When a similar treatment was performed with Ptc DN expressing HCT8, 5E1 antibody was not associated with a tumor growth inhibition -i.e., a non significant tumor growth enhancement- or with a reduction in the net tumor weight (Fig.25EG). Of interest, 5E1 treatment triggers a significant apoptosis -as measured by increased caspase-3 activity- in tumors from mock HCT8 engrafted mice while it had no effect on tumors from Ptc DN expressing HCT8 engrafted mice.

Together, our data support the view that the anti-tumor effect reported after SHH interference is probably not due, as generally considered, to the inhibition of SHH signaling - i.e, Smo-Gli dependent signaling- but to the pro-apoptotic of the dependence receptor Ptc. This can appear in a strong opposition to the general view that proposes that developing therapeutic approaches based on SHH interference and those based on inhibiting the downstream SHH signaling work similarly on inactivating the Ptc-Smo-Gli pathway (Scales 2009, Tremblay 2009). This is also in contradiction with previous works that have shown that drugs acting downstream of Ptc like cyclopamine show anti-tumor effects in tumor cell lines with SHH up-regulation (Berman 2003). However, based on the general toxicity of these compounds, it has been impossible to exclude on off-target effect of these compounds. Moreover a recent work has shown that in settings of SHH high tumors, Smo-Gli signaling is only active in stoma cells but is off in tumor cells (Yauch 2008). Our work thus suggests that candidate drugs targeting SHH signaling should have poor cytotoxic effect on tumors except on tumors showing mutation in the SHH pathway -e.g., increase of SHH signaling through Smo or Gli mutation-. Even though it can be excluded at this point that in some tumor context, inhibition of downstream SHH signaling may show anti-cancer effect in SHH high tumor, our work here propose that in patients with SHH high tumor, interfering with SHH either via SHH mRNA interference, anti-SHH monoclonal antibody or other biologics interfering with SHH/Ptc interaction should have a potent anti-cancer effect due to the release of unbound Ptc pro-apoptotic activity.

### Material and Methods:

### Cell lines and Transfection Procedures:

Human colon cancer cell line HCT8 were cultured in RPMI 1640 Glutamax medium (Gibco ; Invitrogen) containing 10% horse serum. HCT8 cells were transfected by using Lipofectamine reagent (Invitrogen) for plasmids or Lipofectamine 2000 reagent (Invitrogen) for small interfering RNA (siRNA). Puromycin (Sigma) was used to select stable cells at the concentration of 1 µg/ml.

### Plasmid Constructs, siRNA and Shh-neutralizing 5e1 antibody production:

In order to use puromycin to establish stable HCT8 cell lines, we cloned ptcDN in peak8 vector (clontech). Peak8-ptcDN plasmid was obtained by inserting a HindIII/EcoRV fragment generated by polymerase chain reaction performed on the already described pcDNA3-PtcDN (Thibert et al., 2003). HCT8 cell line were then transfected with an empty plasmid (peak8 vector) or peak8-PtcDN and was treated with puromycin 48h after transfection. Shh and Control siRNA were designed by Santa Cruz as q pool of three target-specific siRNAs of 20-25 nucleotides. SHH-neutralizing 5e1 monoclonal antibody were purified from ascites on protein G sepharose column. Then this antibody was concentrated with centricon and change buffer. IgG1 control antibodies were purchase from R&D System.

### Immunoblotting analysis:

Immunoblots were performed as already described (Mille et al. 2009) using anti-Ptc (generously given by M. Ruat) or anti-SHH (R&D system) antibodies.

### Quantitative Reverse Transcription-Polymerase Chain Reaction:

To assay SHH, Dral/FHL2 and Ptc expression, total RNA was extracted from cell lines using the NucleoSpin RNAII kit and 1 µg was reverse transcribed using the iScript cDNA Synthesis kit (Bio-Rad Laboratories). Real-time Q-RT-PCR was performed as previously described (Delloye-Bourgeois et al. 2009b). The ubiquitously expressed human PGK genes showing the least variability in expression in colon cells was used as an internal control.

### Caspase-3 activity assay:

For cell death assays, 1.8.10⁵ cells were cultured in medium without serum and were treated (or not) with SHH-neutralizing 5e1 antibody (1 µg/mL) or transfected with siRNA with Lipofectamine 2000 (Invitrogen). Apoptosis was monitored by measuring caspase-3 activity as described previously (Delloye-Bourgeois et al. 2009b), by using of the Caspase-3/CPP32 Fluorometric Assay Kit (Gentaure Biovision, Brussels, Belgium).

### Chicken model for tumor progression and dissemination:

107 HCT8 cells suspended in 20 µl of PBS mixed with 20 µl of Matrigel were seeded on 10-d-old chick CAM. 10 µg of IgG control or 5e1 antibodies was injected in the tumor on days 11 and 13. On day 17, tumors were resected and primary tumor size and metastasis in lungs were analyzed as previously described (Delloye-Bourgeois et al. 2009a).

### Xenograft of human cell lines in nude mice:

Five-week-old (20 - 22 g [body weight]) female athymic nu/nu mice were obtained from Charles River. The mice were housed in sterilized filter-topped cages and maintained in a pathogen-free animal facility. HCT8 Control or ptcDN HCT8 cells were implanted by subcutaneous injection of 5.10 ⁶ cells in 50 µl of PBS mixed to 50 µl of Matrigel into the left flank of the mice to make one tumor per mouse. When tumors reached a volume of approximately 70 mm³ in approximately 10 days after injection, 300 µg of SHH neutralizing antibody or an equal volume of buffer was injected 3 times per week for 21 days. In other experiments, 3 µg of siRNA targeting SHH or control siRNA was injected intraperitoneally 3 times per week for 9 days. Tumor sizes were measured with a caliper. The tumor volume was calculated with the formula v = 0.5 x (length x width²)..

### References

Ahlgren, S.C., and Bronner-Fraser, M. (1999). Curr Biol 9, 1304-1314.
Berman, D.M., et al. (2003). Nature 425, 846-851.
Bernet, A., et al. (2007). Gastroenterology 133, 1840-1848.
Brito, J.M., et al. (2006). Proc Natl Acad Sci U S A 103, 11607-11612.
Brito, J.M., et al. (2008). Development 135, 2311-2319.
Charrier, J.B., et al. (2001). Development 128, 4011-4020.
Cole, F., and Krauss, R.S. (2003). Cdon. Curr Biol 13, 411-415.
Dahmane, N., et al. (1997). Nature 389, 876-881.
Furne, C.,et al. (2006). Proc Natl Acad Sci U S A 103, 4128-4133.
Furne, C., et al. (2008). Proc Natl Acad Sci U S A 105, 14465-14470.
Ghoumari, A.M.,et al. (2000). Eur J Neurosci 12, 2935-2949.
Goldschneider, D., and Mehlen, P. (2010). Oncogene 29(13) : 1865-82.
Grady, W.M. (2007). Gastroenterology 133, 2045-2049.
Ingham, P.W., and McMahon, A.P. (2001). Genes Dev 15, 3059-3087.
Jessell, T.M. (2000). Nat Rev Genet 1, 20-29.
Kang, J.S., et al. (2002). EMBO J 21, 114-124.
Kang, J.S., et al. (1998). J Cell Biol 143, 403-413.
Kang, J.S.,et al. (2004). J Cell Biol 167, 493-504.
Le Douarin, N.M., and Halpern, M.E. (2000). Curr Opin Neurobiol 10, 23-30*.*
Maisse, C.,et al. (2008). Exp Cell Res 314, 2544-2552.
Martinelli, D.C., and Fan, C.M. (2007).. Genes Dev 21, 1231-1243.
Matsunaga, E., et al. (2004). Nat Cell Biol 6, 749-755.
Mazelin, L., et al. (2004). Nature 431, 80-84.
McLellan, J.S.,et al. (2008). Nature 455, 979-983.
Mehlen, P., and Guenebeaud, C. (1020) Curr Opin Oncology 22(1) : 46-54
Mehlen, P., and Puisieux, A. (2006). Nat Rev Cancer 6, 449-458.
Mehlen, P., et al. (1998). Nature 395, 801-804.
Mille, F., et al.. (2009). Nat Cell Biol 11(b): 739-746.
Ming, J.E., and Muenke, M. (1998). Clin Genet 53, 155-163.
Mulieri, P.J., et al. (2000). Dev Dyn 219, 40-49.
Raffel, C., et al. (1997). Cancer Res 57, 842-845.
Scales, S.J., and de Sauvage, F.J. (2009). Trends Pharmacol Sci 30, 303-312.
Tauszig-Delamasure, S., et al. (2007). Proc Natl Acad Sci U S A 104, 13361-13366.
Taylor, M.D., et al. (2002). Nat Genet 31, 306-310.
Tenzen, T., et al. (2006). Dev Cell 10, 647-656.
Thayer, S.P., et al. (2003). Nature 425, 851-856.
Thibert, C., et al. (2003). Science 301, 843-846.
Tremblay, M.R., et al. (2009). Expert Opin Ther Pat 19, 1039-1056.
Wallis, D., and Muenke, M. (2000). Hum Mutat 16, 99-108.
Watkins, D.N., et al. (2003). Nature 422, 313-317.
Xie, J., et al. (1998). Nature 391, 90-92.
Yauch, R.L., et al. (2008). Nature 455, 406-410.
Yuan, Z., et al. (2007). Oncogene 26, 1046-1055.
Zhang, W.,et al. (2006). Dev Cell 10, 657-665.
Fitamant J et al., Proc Natl Acad Sci U S A 2008;105(12):4850-5.
Delloye-Bourgeois C et al., J Exp Med 2009;206(4):833-47 (2009a)
Delloye-Bourgeois C et al., J Natl Cancer Inst 2009;101 (4):237-47 (2009b)
Bouzas-Rodriguez J et al., J Clin Invest;120(3):850-8.
Paradisi A et al., Proc Natl Acad Sci U S A 2009;106(40):17146-51.
Stupack DG et al., Nature 2006;439(7072):95-9.

SEQ ID NO :1
CDO Third FbnIII domain
Arg-Phe-Ser-Ser-Arg-Pro-Ile-Thr-Gly-Pro-His-Ile-Ala-Tyr-Thr-Glu-Ala-Val-Ser-Asp-Thr-Gln-
Ile-Met-Leu-Lys-Trp-Thr-Tyr-Ile-Pro-Ser-Ser-Asn-Asn-Asn-Thr-Pro-Ile-Gln-Gly-Phe-Tyr-Ile-
Tyr-Tyr-Arg-Pro-Thr-Asp-Ser-Asp-Asn-Asp-Ser-Asp-Tyr-Lys-Arg-Asp-Val-Val-Glu-Gly-Ser-
Lys-Gln-Trp-His-Met-Ile-Gly-His-Leu-Gln-Pro-Glu-Thr-Ser-Tyr-Asp-Ile-Lys-Met-Gln-Cys-Phe-
Asn-Glu-Gly-Gly-Glu-Ser

SEQ ID NO :2
IgG Fc fragment
Asp-Lys-Thr-His-Thr-Cys-Pro-Pro-Cys-Pro-Ala-Pro-Glu-Leu-Leu-Gly-Gly-Pro-Ser-Val-Phe-
Leu-Phe-Pro-Pro-Lys-Pro-Lys-Asp-Thr-Leu-Met-Ile-Ser-Arg-Thr-Pro-Glu-Val-Thr-Cys-Val-
Val-Val-Asp-Val-Ser-His-Glu-Asp-Pro-Glu-Val-Lys-Phe-Asn-Trp-Tyr-Val-Asp-Gly-Val-Glu-
Val-His-Asn-Ala-Lys-Thr-Lys-Pro-Arg-Glu-Glu-Gln-Tyr-Asn-Ser-Thr-Tyr-Arg-Val-Val-Ser-Val-
Leu-Thr-Val-Leu-His-Gln-Asp-Trp-Leu-Asn-Gly-Lys-Glu-Tyr-Lys-Cys-Lys-Val-Ser-Asn-Lys-
Ala-Leu-Pro-Ala-Pro-Ile-Glu-Lys-Thr-Ile-Ser-Lys-Ala-Lys-Gly-Gln-Pro-Arg-Glu-Pro-Gln-Val-
Tyr-Thr-Leu-Pro-Pro-Ser-Arg-Glu-Glu-Met-Thr-Lys-Asn-Gln-Val-Ser-Leu-Thr-Cys-Leu-Val-
Lys-Gly-Phe-Tyr-Pro-Ser-Asp-Ile-Ala-Val-Glu-Trp-Glu-Ser-Asn-Gly-Gln-Pro-Glu-Asn-Asn-
Tyr-Lys-Thr-Thr-Pro-Pro-Val-Leu-Asp-Ser-Asp-Gly-Ser-Phe-Phe-Leu-Tyr-Ser-Lys-Leu-Thr-
Val-Asp-Lys-Ser-Arg-Trp-Gln-Gln-Gly-Asn-Val-Phe-Ser-Cys-Ser-Val-Met-His-Glu-Ala-Leu-
His-Asn-His-Tyr-Thr-Gln-Lys-Ser-Leu-Ser-Leu-Ser-Pro-Gly-Lys-

SEQ ID NO :3
Peptide Signal: Kappa2 peptide signal
Met-Asp-Phe-Gly-Leu-Arg-Leu-Ile-Phe-Leu-Val-Leu-Val-Leu-Lys-Gly-Val-Leu-Cys

## Claims

1. The use of an effective amount of an agonist of the CDO's apoptotic function for the preparation of a drug to induce cell apoptosis in a patient having tumoral cells bearing CDO and expressing SHH.

2. The use according to claim 1, wherein the agonist is a CDO fragment, a fusion protein comprising a CDO fragment, an antibody against SHH, or a siRNA which is capable of inhibiting SHH expression.

3. The use of an effective amount of agonists of the CDO's and PTC1 apoptotic functions for the preparation of a drug to induce cell apoptosis in a patient having tumoral cells bearing CDO and PTC1 and expressing SHH.

4. Fusion protein containing a CDO fragment which specifically bind to Sonic Hedgehog (SHH), for use as an agonist of the CDO's apoptotic function as a therapeutic agent to induce apoptosis of tumoral cells in a patient having tumoral cells bearing CDO and expressing SHH.

5. Fusion protein according to claim 4, for use as an agonist of the CDO's apoptotic function as a therapeutic agent to induce apoptosis of tumoral cells in a patient with autocrine or paracrine SHH expression.

6. Fusion protein according to claim 4 or 5, for use as an agonist of the CDO's apoptotic function as a therapeutic agent to induce apoptosis of non small cell lung cancer in a patient.

7. Fusion protein according to any one of claims 4 to 6, wherein the CDO fragment comprises CDO FNIII (3), CDO FNIII (1-3), or CDO extracellular domain.

8. Fusion protein according to any one of claims 4 to 7, comprising an amino acid sequence as depicted on SEQ ID NO:1 and/or an amino acid sequence from an IgG, and/or the amino acid sequence of a Fc fragment.

9. Fusion protein according to claim 8, wherein the Fc comprises sequence SEQ ID NO:2.

10. Anti-SHH antibody, for use as an agonist of the CDO's apoptotic function as a therapeutic agent to induce apoptosis of tumoral cells in a patient having tumoral cells bearing CDO and expressing SHH.

11. Antibody according to claim 10, for use as an agonist of the CDO's apoptotic function as a therapeutic agent to induce apoptosis of tumoral cells in a patient with autocrine or paracrine SHH expression, or of non small cell lung cancer in a patient.

12. siRNA which is capable of inhibiting SHH expression, for use as an agonist of the CDO's apoptotic function as a therapeutic agent to induce apoptosis of tumoral cells in a patient having tumoral cells bearing CDO and expressing SHH..

13. siRNA according to claim 12, for use as an agonist of the CDO's apoptotic function as a therapeutic agent to induce apoptosis of tumoral cells in a patient with autocrine or paracrine SHH expression.

14. siRNA according to claim 12 or 13, for use as an agonist of the CDO's apoptotic function as a therapeutic agent to induce apoptosis of non small cell lung cancer in a patient.

15. Pharmaceutical composition containing an agonist of the CDO's apoptotic function or a CDO polypeptide or an antibody specific to CDO, and an agonist of the PTC1's apoptotic function or a PTC1 polypeptide or an antibody specific to PTC1, and a pharmaceutically acceptable excipient or vehicle.
